# FASCICULE DE BREVET EUROPEEN

(11) **EP 3 948 273 B1**
(45) Date de publication et mention de la délivrance du brevet: **03.07.2024**
(21) Numéro de dépôt: 20713341.4
(22) Date de dépôt: 30.03.2020
(51) Int. Cl.: G01N 33/487, G01N 11/14

(54) **MÉTHODE IN VITRO DE DIAGNOSTIC, DE STRATIFICATION, DE PRONOSTIC ET/OU DE SUIVI D'UNE MALADIE DES POUMONS ET/OU UNE MALADIE RESPIRATOIRE**
IN-VITRO-VERFAHREN ZUR DIAGNOSE, STRATIFIZIERUNG, PROGNOSE UND/ODER ÜBERWACHUNG EINER LUNGENERKRANKUNG UND/ODER EINER ATEMWEGSERKRANKUNG
IN VITRO METHOD FOR DIAGNOSING, STRATIFYING, PROGNOSING AND/OR MONITORING A LUNG DISEASE AND/OR A RESPIRATORY DISEASE

(30) Priorité: 28.03.2019 FR 1903302
(43) Date de publication de la demande: 09.02.2022
(73) Titulaire: Rheonova, 38610 Gières (FR)
(72) Inventeur: PATARIN, Jérémy, 38420 REVEL (FR)
(74) Mandataire: Regimbeau
(86) Numéro de dépôt international: PCT/EP2020/058982
(87) Numéro de publication internationale: WO 2020/193811

(56) Documents cités:
- JEREMY PATARIN ET AL: "PA5051: Change of mucus rheology in patients with Cystic Fibrosis, COPD and Asthma", EUROPEAN RESPIRATORY JOURNAL, vol. 52, no. Suppl. 62, 15 septembre 2018 (2018-09-15), page PA5051, XP055660824, GB ISSN: 0903-1936, DOI: 10.1183/13993003.congress-2018.PA5051
- GIOVANNA TOMAIUOLO ET AL: "A New Method to Improve the Clinical Evaluation of Cystic Fibrosis Patients by Mucus Viscoelastic Properties", PLOS ONE, vol. 9, no. 1, 3 janvier 2014 (2014-01-03) , page e82297, XP055661732, DOI: 10.1371/journal.pone.0082297
- C.J. NETTLE ET AL: "Linear rheology as a potential monitoring tool for sputum in patients with Chronic Obstructive Pulmonary Disease (COPD)", BIORHEOLOGY., vol. 54, no. 2-4, 2 octobre 2017 (2017-10-02), pages 67-80, XP055661382, GB ISSN: 0006-355X, DOI: 10.3233/BIR-17141
- SERISIER DAVID J ET AL: "Macrorheology of cystic fibrosis, chronic obstructive pulmonary disease & normal sputum", RESPIRATORY RESEARCH, BIOMED CENTRAL LTD., LONDON, GB, vol. 10, no. 1, 6 juillet 2009 (2009-07-06), page 63, XP021055543, ISSN: 1465-9921, DOI: 10.1186/1465-9921-10-63
- Anonymous: "Understanding Yield Stress Measurements", , 4 août 2015 (2015-08-04), XP055705127, Extrait de l'Internet: URL:https://cdn.technologynetworks.com/TN/ Resources/PDF/WP120416UnderstandYieldStres sMeas.pdf [extrait le 2020-06-15]

## Description

### DOMAINE DE L'INVENTION

La présente invention se situe dans le domaine du diagnostic et du suivi de maladies des poumons et/ou de maladies respiratoires. Elle concerne une méthode *in vitro* de diagnostic, de stratification, de pronostic et/ou de suivi d'une maladie des poumons et/ou une maladie respiratoire, comprenant la détermination ou la mesure d'au moins un paramètre rhéologique sur un échantillon de mucus/d'expectoration d'un sujet, dans la zone non linéaire de la courbe de déformation de l'échantillon et d'au moins un paramètre rhéologique dans la zone linéaire de ladite courbe, ainsi que le diagnostic, la stratification, le pronostic et/ou le suivi de la maladie des poumons et/ou de la maladie respiratoire à partir d'une comparaison desdites valeurs Anl et Al déterminées, avec une valeur limite respective. La présente invention concerne également une méthode *in vitro* d'évaluation de l'efficacité d'un traitement d'une maladie des poumons et/ou d'une maladie respiratoire, basée sur la détermination ou la mesure desdits paramètres. La présente invention est telle que définie dans les revendications.

### ETAT DE LA TECHNIQUE

L'appareil respiratoire est constitué d'organes particulièrement sensibles aux infections et aux traumatismes (en particulier les poumons), de par leur grande surface de contact et leur constante exposition aux particules, aux produits chimiques et aux organismes infectieux présents dans l'air ambiant.

Les maladies respiratoires et les maladies des poumons sont des maladies pouvant avoir des conséquences graves et causant de nombreux décès dans toutes les régions du globe.

Elles sont pourtant souvent non diagnostiquées ou peuvent être diagnostiquées tardivement, lorsque la maladie est à un stade avancé et plus difficile à traiter.

Selon un rapport publié par l'Organisation Mondiale de la Santé (OMS) en 2017, les estimations portent à 65 millions le nombre de personnes souffrant de bronchopneumopathie chronique obstructive (BPCO) au niveau mondial, une maladie qui cause 3 millions de décès par an. Environ 334 millions de personnes dans le monde sont asthmatiques, avec une prévalence sans cesse croissante chez les enfants, tandis que des millions d'autres souffraient de rhinite allergique et d'autres maladies respiratoires chroniques, rarement diagnostiquées et parfois mortelles (The Global Impact of Respiratory Diseases, Second Edition, Forum of International Respiratory Societies, 2017).

Les principaux symptômes conduisant à une recherche de maladies respiratoires ou de maladies des poumons sont une gêne respiratoire et une expectoration anormale.

Les outils de diagnostic et de suivi des patients comprennent notamment les tests d'épreuves fonctionnelles respiratoires (consistant à mesurer la capacité des poumons à retenir et déplacer l'air et à échanger l'oxygène et le dioxyde de carbone), les analyses microbiologiques des expectorations, les examens d'imagerie, la bronchoscopie et la thoracoscopie.

Toutefois, ces méthodes sont peu adaptées à un diagnostic précoce et la discrimination des différentes maladies ou de leur stade d'évolution (stratification) reste difficile et totalement empirique, ce qui peut conduire à des traitements inadaptés ou trop tardifs.

L'expectoration est un facteur essentiel du dégagement normal des voies respiratoires et de la fonction pulmonaire. On retrouve le mucus dans les voies aériennes supérieures où il forme une couche protectrice de l'épithélium. Le mucus aide à maintenir l'homéostasie des voies respiratoires en aidant à l'élimination des agents pathogènes et des particules étrangères. Un équilibre dans la production de mucus et la clairance est essentiel à la santé des voies respiratoires et les changements dans les propriétés viscoélastiques peuvent grandement affecter ce processus.

Les propriétés viscoélastiques du mucus ont été étudiées chez des patients souffrant de broncho pneumopathie chronique obstructive (BPCO) ou de mucoviscidose, sur la base des modules d'élasticité (G') et de viscosité (G"). Différentes études suggèrent que ces propriétés viscoélastiques évoluent en fonction du stade de la maladie, chez des patients souffrant de la BPCO ou de la mucoviscidose (Serisier et al., 2009 ; WO2011/121462; Tomaiuolo et al., 2014 ; Nettle et al., 2017 ; Ma et al., 2018). Toutefois, les données n'ont pas permis de discriminer ces différentes maladies respiratoires de manière statistique. En outre, les mesures sont effectuées dans des conditions opératoires quasi-statiques qui ne sont pas représentatives du mucus en conditions physiologiques. De ce fait, ces mesures restent peu fiables et ne permettent pas d'analyser ou de prédire assez finement l'état du patient.

Patarin et al., 2018, ERJ, XP055660824, décrit une étude clinique réalisée sur des patients souffrant de BPCO, de mucoviscidose ou d'asthme. Des propriétés rhéologiques, incluant la viscosité, l'élasticité et la plasticité, de mucus provenant de ces patients ont été mesurées. Cependant, aucune évaluation du module complexe G* ou de la contrainte critique σ_{c} n'a été effectuée.

Il existe donc le besoin de développer de nouveaux tests diagnostiques des maladies respiratoires et de maladies des poumons, basés sur des mesures des propriétés rhéologiques du mucus en conditions physiologiques, permettant ainsi la prédiction fiable de l'état du patient, pour favoriser une prise en charge plus précoce ainsi que la mise et la mise en place de traitements ciblés.

La présente invention permet de répondre à ce besoin. Les présents Inventeurs ont mené trois études cliniques ayant permis de démontrer pour la première fois que les paramètres rhéologiques du mucus de patients atteints de différentes maladies respiratoires sont statistiquement différents d'une maladie à l'autre, permettant ainsi de distinguer les différentes maladies respiratoires et pulmonaires. Les Inventeurs ont notamment montré que ces paramètres rhéologiques peuvent être utilisés comme des biomarqueurs et permettent de discriminer les maladies respiratoires à la fois lorsqu'ils sont mesurés dans la zone linéaire de la courbe de déformation du mucus et à la fois lorsqu'ils sont mesurés dans la zone non linéaire de cette courbe. Les données montrent que la combinaison de la détermination ou la mesure d'un paramètre rhéologique dans la zone linéaire avec la détermination ou la mesure d'un paramètre rhéologique (identique ou différent) dans la zone linéaire de la courbe de déformation du mucus permet une discrimination particulièrement fiable, efficace et significative. De plus, les données montrent que le traitement préalable de patients à la rhDNase (désoxyribonucléase recombinante humaine) a un effet sur les paramètres rhéologiques du mucus du patient, révélant ainsi pour la première fois que la mesure de paramètres rhéologiques permet d'évaluer l'effet mucolytique d'un traitement.

Les données obtenues par les inventeurs sont d'autant plus pertinentes qu'elles ont été obtenues à l'aide d'un dispositif mis au point par les Inventeurs, permettant de simuler l'écoulement du mucus en situation physiologique. Les Inventeurs ont notamment montré que ce dispositif permet d'obtenir de manière reproductible des mesures des propriétés viscoélastiques et rhéologiques du mucus placé en conditions physiologiques. Les Inventeurs ont ainsi mis au point une nouvelle méthode permettant un diagnostic, une stratification, un pronostic et un suivi fiable des maladies des poumons et des maladies respiratoires, basée sur la mesure de paramètres rhéologiques dans la zone linéaire de la courbe de déformation et de paramètres rhéologiques dans la zone non linéaire de cette courbe obtenue à partir du mucus de patients. Les Inventeurs ont également mis au point une nouvelle méthode d'évaluation de l'efficacité d'un traitement de maladies des poumons et de maladies respiratoires, basée sur la mesure de ces paramètres.

Cette invention fournit ainsi de nouveaux tests diagnostiques, tels des tests compagnons, permettant la prédiction fiable de l'état du patient et la mise en place de traitements ciblés.

### EXPOSE DE L'INVENTION

Dans le cadre de la présente invention, les Inventeurs ont mis en évidence, de manière tout à fait surprenante, que la détermination ou la mesure d'une combinaison de paramètres rhéologiques sur le mucus de patients, à la fois dans la zone linéaire et dans la zone non linéaire de la courbe de déformation du mucus, permet de distinguer différentes maladies pulmonaires et/ou respiratoires.

La présente invention est telle que définie dans les revendications.

La présente invention concerne donc notamment une nouvelle méthode *in vitro* de diagnostic, de stratification, de pronostic et/ou de suivi d'une maladie des poumons et/ou une maladie respiratoire comprenant la détermination ou la mesure de tels paramètres rhéologiques, ainsi que le diagnostic, la stratification, le pronostic et/ou le suivi de la maladie des poumons et/ou de la maladie respiratoire à partir d'une comparaison desdites valeurs Anl et Al déterminées, avec une valeur limite respective. L'invention concerne également une nouvelle méthode *in vitro* d'évaluation de l'efficacité d'un traitement d'une maladie des poumons et/ou d'une maladie respiratoire, comprenant la détermination ou la mesure de tels paramètres rhéologiques, ainsi que le diagnostic, la stratification, le pronostic et/ou le suivi de la maladie des poumons et/ou de la maladie respiratoire à partir d'une comparaison desdites valeurs Anl et Al déterminées, avec une valeur limite respective. Il est également ici décrit l'utilisation de tels paramètres rhéologiques pour diagnostiquer, stratifier, pronostiquer et/ou suivre une maladie des poumons et/ou une maladie respiratoire ; et/ou pour évaluer l'efficacité d'un traitement d'une maladie des poumons et/ou d'une maladie respiratoire, qui ne figurent pas dans le texte des revendications.

Il est également ici décrit un test compagnon, utilisant de tels paramètres rhéologiques pour diagnostiquer, stratifier, pronostiquer et/ou suivre une maladie des poumons et/ou une maladie respiratoire ; et/ou pour évaluer l'efficacité d'un traitement d'une maladie des poumons et/ou d'une maladie respiratoire, qui ne figurent pas dans le texte des revendications.

### DESCRIPTION DES FIGURES

La **Figure 1** présente un exemple type de courbe de déformation et montre la zone linéaire, qui comprend un plateau, et la zone non linéaire.
La **Figure 2** représente un graphique montrant la moyenne des valeurs obtenues pour le paramètre G' mesuré à 5% de déformation, chez des sujets sains ou des patients souffrant de mucoviscidose (Muco), de BPCO ou d'asthme, mesurées à t1 (noir) et t2= t1 +10 minutes (gris).
La **Figure 3** est un graphique montrant la moyenne des valeurs obtenues pour le paramètre G' mesuré au point d'écoulement c (**G**'_{c}), chez des sujets sains ou des patients souffrant de mucoviscidose (Muco), de BPCO ou d'asthme, mesurées à t1 (noir) et t2= t1+10 minutes (gris).
La **Figure 4** **est** un graphique montrant la moyenne des valeurs obtenues pour le paramètre G" mesuré à 5% de déformation, chez des sujets sains ou des patients souffrant de mucoviscidose (Muco), de BPCO ou d'asthme, mesurées à t1 (noir) et t2= t1+10 minutes (gris).
La **Figure 5** est un graphique montrant la moyenne des valeurs obtenues pour le paramètre tan δ déterminé dans le domaine linéaire, chez des sujets sains ou des patients souffrant de mucoviscidose (Muco), de BPCO ou d'asthme, déterminées à t1 (noir) et t2= t1+10 minutes (gris).
La **Figure 6** est un graphique montrant la moyenne des valeurs obtenues pour le paramètre τ mesuré à la limite supérieure du domaine linéaire (τₚ) (correspondant à la zone de fin du plateau de la courbe de déformation), chez des sujets sains ou des patients souffrant de mucoviscidose (Muco), de BPCO ou d'asthme, mesurées à t1 (noir) et t2= t1+10 minutes (gris).
La **Figure 7** est un graphique montrant la moyenne des valeurs obtenues pour le paramètre σ mesuré au point d'écoulement c (σ_{c}), chez des sujets sains ou des patients souffrant de mucoviscidose (Muco), de BPCO ou d'asthme, mesurées à t1 (noir) et t2= t1+10 minutes (gris).
La **Figure 8** est un graphique montrant la moyenne des valeurs obtenues pour le paramètre γ correspondant à la limite supérieure du domaine linéaire (γₚ) (correspondant à la zone de fin du plateau), chez des sujets sains ou des patients souffrant de mucoviscidose (Muco), de BPCO ou d'asthme, mesurées à t1 (noir) et t2= t1 +10 minutes (gris).
La **Figure 9** est un graphique montrant la moyenne des valeurs obtenues pour le paramètre γ mesuré au point d'écoulement c (γ_{c}), chez des sujets sains ou des patients souffrant de mucoviscidose (Muco), de BPCO ou d'asthme, mesurées à t1 (noir) et t2= t1+10 minutes (gris).
La **Figure 10** est un graphique montrant la moyenne des valeurs obtenues pour le paramètre EF, déterminé chez des sujets sains ou des patients souffrant de mucoviscidose (Muco), de BPCO ou d'asthme, déterminées à t1 (noir) et t2= t1 +10 minutes (gris).
La **Figure 11** est un graphique montrant les valeurs des paramètres G' et G" mesurés à 5% de déformation et des paramètres G'_{c} et σ_{c} mesurés au point d'écoulement chez des patients atteints de mucoviscidose, avant (noir) et après (gris) traitement à la rhDNase.
La **Figure 12** est un graphique montrant les valeurs du paramètre σ_{c} mesurés chez des patients atteints de dilatation des bronches (DDB), de BPCO ou d'asthme lors des deux études cliniques n°2 et 3 (menées respectivement par le CHU de Montpellier (losanges gris) et par l'hôpital universitaire de Bâle (carrés noirs)), ainsi que la limite de détection des 3 maladies (ligne grise) et la limite de détection d'une exacerbation (ligne noire), impliquant une décision thérapeutique.

### DESCRIPTION DETAILLEE DE L'INVENTION

### Définitions

Par « **diagnostic** », on entend l'identification/la détermination d'une maladie, ou de l'absence d'une maladie, chez un sujet. Le diagnostic comprend par exemple la recherche des causes (étiologie) et des effets (symptômes) de la maladie, notamment sur la base d'observations et/ou de mesures, effectuées à l'aide de différents outils. Dans le cas des maladies respiratoires et/ou pulmonaires, les outils de diagnostic comprennent les tests d'épreuves fonctionnelles respiratoires (consistant à mesurer la capacité des poumons à retenir et déplacer l'air et à échanger l'oxygène et le dioxyde de carbone), les analyses microbiologiques des expectorations, les examens d'imagerie, la bronchoscopie et la thoracoscopie.

Par « **stratification** », on entend la séparation/classification de sujets en sous-groupes par sévérité/gravité de la maladie. Les différents sous-groupes comprennent notamment le sous-groupe des sujets sains ainsi que différents sous-groupes de sujets souffrant d'une maladie, classés en fonction du stade d'évolution/d'avancement de la maladie. Le stade d'évolution peut être déterminé sur la base d'observations et/ou de mesures, effectuées à l'aide de différents outils. Dans le cas des maladies respiratoires et/ou pulmonaires, les outils de stratification comprennent les outils qui sont également utilisés pour leur diagnostic.

Par « **pronostic** », on entend la prédiction/la détermination/l'évaluation des risques d'évolution d'une maladie chez un sujet. Le pronostic comprend notamment l'évaluation du développement futur de l'état du sujet et des chances éventuelles de guérison. Le pronostic peut être déterminé sur la base d'observations et/ou de mesures, effectuées à l'aide de différents outils. Dans le cas des maladies respiratoires et/ou pulmonaires, les outils de pronostic comprennent les outils qui sont également utilisés pour leur diagnostic ou la stratification des sujets.

Par « **suivi** », on entend la détermination/l'évaluation de l'évolution d'une maladie chez un sujet. Le suivi peut être réalisé sur la base d'observations et/ou de mesures, effectuées à l'aide de différents outils, à différents intervalles de temps. Les intervalles peuvent être réguliers ou irréguliers. Leur fréquence dépend de la maladie mais aussi du stade d'évolution de la maladie. Elle peut être de l'ordre de quelques jours (par exemple en cas de maladie à un stade sévère/avancé/grave et/ou en cas de maladie à évolution rapide et/ou en cas de phase d'exacerbation) à quelques années (par exemple en cas de maladie à un stade préliminaire, léger ou modéré, et/ou en cas de maladie à évolution lente). Dans le cas des maladies respiratoires et/ou pulmonaires, les outils de suivi comprennent les outils qui sont également utilisés pour le diagnostic ou le pronostic de la maladie, ou la stratification des sujets.

Par « **évaluation de l'efficacité d'un traitement** », on entend la détermination de l'état clinique d'un sujet soumis à un traitement. Le traitement peut être préventif, par exemple en cas de prédisposition à une maladie, ou il peut être curatif, par exemple en cas de maladie diagnostiquée. L'efficacité du traitement peut par exemple être évaluée en déterminant l'état du sujet à différents intervalles de temps. L'état du sujet peut notamment être évalué avant la première prise du traitement puis à des intervalles de temps réguliers (ou irréguliers) après cette première prise (par exemple après chaque nouvelle prise du traitement). Une comparaison de l'état du sujet évalué à ces différents intervalles peut alors être effectuée afin d'identifier un éventuel changement. L'état du patient peut être évalué sur la base d'observations et/ou de mesures, effectuées à l'aide de différents outils. Dans le cas des maladies respiratoires et/ou pulmonaires, les outils d'évaluation de l'efficacité d'un traitement comprennent les outils qui sont également utilisés pour le diagnostic, le pronostic ou le suivi de la maladie, ou la stratification des sujets.

Par « **test compagnon** », on entend un test diagnostic permettant de sélectionner, en fonction de leur statut pour un marqueur identifié par ce test (tel qu'un marqueur prédictif), uniquement les patients chez lesquels le traitement est susceptible d'apporter un bénéfice parmi ceux diagnostiqués pour une maladie donnée. Le test compagnon est par exemple tel que défini par la Haute Autorité de la Santé (notamment tel que défini dans le Guide Méthodologique « Test compagnon associé à une thérapie ciblée : définitions et méthode d'évaluation » publié en février 2014. Le marqueur est de préférence la valeur d'un paramètre rhéologique, de préférence encore la valeur d'un paramètre rhéologique déterminée/mesurée sur un échantillon de mucus d'un sujet, ledit paramètre rhéologique étant avantageusement tel que défini ci-dessous et/ou étant déterminé/mesuré tel qu'expliqué ci-dessous.

Par « **maladie respiratoire** », on entend une maladie (ou affection) affectant l'appareil respiratoire. L'appareil respiratoire est un ensemble d'organes permettant la respiration (c'est-à-dire les échanges gazeux entre l'organisme et l'environnement). Chez les mammifères tels que l'humain, l'appareil respiratoire comprend le nez, la bouche, le pharynx, le larynx, la trachée, le diaphragme et les poumons, ces derniers comprenant les bronches, les bronchioles, les alvéoles et les acini. Par « **maladie des poumons** » ou « **maladie pulmonaire** », on entend une maladie respiratoire qui affecte les poumons. Les maladies respiratoires et/ou pulmonaires peuvent être d'origine infectieuse, virale, génétique environnementale, ou résulter d'une combinaison de ces facteurs. Les maladies respiratoires et les maladies des poumons comprennent par exemple l'amiantose ; l'apnée du sommeil ; le syndrome d'apnées obstructives du sommeil ; l'asphyxie ; l'asthme ; la bronchiectasie ; la bronchite, dont la bronchite aiguë et la bronchite chronique ; la broncho pneumopathie chronique obstructive (BPCO) ; le cancer du poumon ; le cancer bronchique ; le carcinome pulmonaire ; la tumeur pulmonaire ; la coqueluche ; le croup ; le déficit en alpha1-antitrypsine ; l'emphysème ; la mucoviscidose ; la fibrose pulmonaire idiopathique ; la grippe, dont la grippe saisonnière et la grippe A ; le syndrome pulmonaire à hantavirus ; l'hypertension artérielle pulmonaire (HTAP) ; la lymphangioléiomyomatose (LAM) ; la maladie pulmonaire obstructive chronique (MPOC) ; la pleurésie ; la pneumonie ; la bronchopneumonie ; l'embolie pulmonaire ; l'œdème pulmonaire ; l'abcès pulmonaire ; le rhume ; la sinusite ; la sarcoïdose ; le traumatisme thoracique ; la toux chronique ; la tuberculose ; l'infection par le virus respiratoire syncytial (VRS) ; la bronchiolite ; la bronchiolite oblitérante avec organisation pneumonique (BOOP) ; l'allergie, en particulier l'allergie respiratoire ; la rhinite allergique ; la dyskinésie ciliaire primitive (DCP) ; la dilatation des bronches (DDB) ; la pneumoconiose ; le pneumothorax (PNO) ; la pleurésie ; le cancer de la plèvre ; la légionellose ; la psittacose ; de préférence ladite maladie est choisie parmi la DDB, la BPCO, la mucoviscidose et l'asthme.

**Par « stade d'une maladie respiratoire et/ou pulmonaire » « stade d'évolution d'une maladie respiratoire et/ou pulmonaire » ou « stade d'avancement d'une maladie respiratoire et/ou pulmonaire** », on entend une phase de la maladie respiratoire et/ou pulmonaire qui est déterminée selon la gravité des symptômes dont souffrent le sujet et leurs implications/conséquences sur le mode et/ou la qualité de vie du sujet. Ces stades peuvent être au nombre de quatre. Par exemple :
- Au stade 1 (ou premier stade), on parle de maladie légère ou de stade léger : le malade commence notamment à présenter une dyspnée (essoufflement à l'effort), encore limitée aux efforts importants, ce qui explique qu'il n'en ait souvent pas conscience et se contente de s'adapter en limitant ses activités.
- Au stade 2 (ou deuxième stade), on parle de maladie modérée ou de stade modéré : l'essoufflement devient présent dans des activités de la vie quotidienne et commence à représenter une source de handicap.
- Au stade 3 (ou troisième stade), on parle de maladie sévère ou de stade sévère : on retrouve les mêmes symptômes qu'au stade 2 mais la dyspnée a lieu aussi pour des efforts minimes de la vie de tous les jours.
- Au stade 4 (ou quatrième stade), on parle de maladie très sévère ou de stade très sévère : le malade est gêné dans les gestes les plus simples (se laver, s'habiller) puis devient insuffisant respiratoire (impossibilité d'assurer pleinement l'oxygénation de son organisme, ce qui se traduit par la cyanose : bleuissement plus ou moins marqué des extrémités des doigts et des lèvres). La qualité de vie est, à ce stade, considérablement altérée. A terme, l'insuffisance respiratoire peut retentir sur le coeur. Des oedèmes des chevilles peuvent apparaître.

Par « **exacerbation** » ou « **phase d'exacerbation** », on entend une période durant laquelle les signes cliniques d'une maladie respiratoire et/ou pulmonaire se majorent chez un sujet souffrant de ladite maladie. Les signes cliniques qui amplifient lors d'une exacerbation comprennent la toux, l'expectoration ainsi que l'essoufflement. L'exacerbation peut être dues à une infection des bronches. L'exacerbation peut se produire à tous les stades de la maladie. Elle est en général peu inquiétante au cours des premiers stades de la maladie mais peut être plus grave quand la maladie est plus avancée. Par exemple, les patients suivis pour BPCO présentent en moyenne près de 2 exacerbations par an.

Par « **sujet** » ou « **patient** », on entend un individu humain ou un animal différent d'un humain. Le sujet est par exemple un humain ou un animal susceptible d'être atteint d'une maladie pulmonaire et/ou respiratoire ou souffrant d'une telle maladie. Le sujet est de préférence un être humain. Le sujet peut être un enfant (sujet humain âgé de 16 ans ou moins) ou un adulte (sujet humain âgé de plus de 16 ans). Par « **sujet sain** » on entend un sujet qui ne souffre pas de la maladie considérée. Dans le cadre de la présente invention, un sujet sain est de préférence un sujet qui ne souffre d'aucune maladie respiratoire et/ou pulmonaire, de préférence encore un sujet qui ne souffre d'aucune maladie.

Par « **mucus** » ou **« expectoration** » ou « **sécrétion broncho-pulmonaire** » ou « **sputum** » ou « **crachat** », on entend une substance plus ou moins visqueuse sécrétée par les glandes muqueuses chez l'humain et l'animal et servant d'enduit protecteur à la surface des muqueuses. L'expectoration est un facteur essentiel du dégagement normal des voies respiratoires et de la fonction pulmonaire, que l'on retrouve dans les voies aériennes supérieures où elle forme une couche protectrice de l'épithélium. Un équilibre dans la production d'expectorations et la clairance est essentielle à la santé des voies respiratoires. Les expectorations comprennent notamment des glycoprotéines de poids moléculaire élevé, dont des mucopolysaccharides par exemple de la mucine. Les glycoprotéines présentes dans le mucus peuvent former une structure polymère réticulée qui détermine, au moins en partie, son comportement viscoélastique. Le mucus peut également comprendre des cellules inflammatoires, des bactéries, de l'actine filamenteuse et de l'ADN hautement polymérisé. Au niveau macroscopique, les expectorations peuvent être considérées comme un gel thixotrope non newtonien, qui se distingue par sa réponse aux contraintes de cisaillement.

Par « **paramètre rhéologique** », on entend une variable dont la valeur est mesurable, permettant de qualifier les propriétés rhéologiques d'un fluide ou d'un solide mou. Ces paramètres sont couramment mesurés et/ou déterminés à l'aide d'un rhéomètre.

Par « **propriété rhéologique** », on entend les propriétés de déformation et d'écoulement d'un fluide ou d'un solide mou, évaluées sous l'effet de contraintes qui lui sont appliquées. Les propriétés de déformation caractérisent la manière dont réagit un matériau donné quand il est soumis à des contraintes mécaniques. **L'écoulement** correspond au mouvement global et irréversible d'un matériau donné. Les propriétés rhéologiques comprennent notamment la viscosité, l'élasticité, la viscoélasticité et la plasticité. Ces propriétés sont couramment caractérisées par des paramètres rhéologiques.

Par « **viscosité** », on entend l'ensemble des phénomènes de résistance à l'écoulement se produisant dans la masse d'une matière, pour un écoulement uniforme et sans turbulence. La viscosité d'un matériau traduit sa capacité à dissiper de l'énergie (par exemple sous forme de chaleur). Plus la viscosité augmente, et plus la capacité du fluide à s'écouler facilement diminue, plus l'énergie dissipée par l'écoulement sera importante.

Par « **élasticité** », on entend la capacité d'un matériau à conserver et restituer de l'énergie après déformation. Un matériau élastique se déforme instantanément sous l'action d'une force exercée et retrouve sa forme et sa taille initiales quand cette force ne s'exerce plus. Un matériau n'est généralement élastique que jusqu'à une certaine limite de la valeur de ces forces.

Par « **viscoélasticité** », on entend la propriété de matériaux qui présentent des caractéristiques à la fois visqueuses et élastiques, lorsqu'ils subissent une déformation. Les matériaux visqueux, résistent à un écoulement en cisaillement et présentent une déformation qui augmente linéairement avec le temps lorsqu'une contrainte est appliquée.

Par « **plasticité** », on entend la propriété d'un matériau de se déformer de manière irréversible. La déformation plastique se produit par un réarrangement local et irréversible de la position relative des atomes, ou plus généralement des éléments constitutifs du matériau. Sous l'effet d'une contrainte, un matériau commence en général par se déformer de manière réversible (déformation élastique), c'est-à-dire que ses dimensions changent et qu'il reprend sa forme initiale lorsque la contrainte s'arrête. Les matériaux dits fragiles cassent dans ce mode de déformation si la contrainte est trop forte. Les matériaux dits ductiles sont déformés de manière définitive, à partir d'un seuil de contrainte : lorsque la contrainte est arrêtée, le matériau reste déformé.

Par « **viscoplasticité** », on entend le comportement inélastique d'un matériau qui dépend de la vitesse de déformation de celui-ci. La dépendance à la vitesse de déformation, dans ce contexte signifie que les déformations sont proportionnelles à la vitesse de chargement. Le comportement inélastique dans le cas de la viscoplasticité est un comportement plastique ce qui signifie que le matériau subit des déformations irréversibles quand un certain niveau de chargement est atteint.

Par « **déformation** » ou « **déformation relative** » ou « y » on entend une modification d'au moins une des dimensions géométriques d'un matériau, relativement à la valeur au repos de ladite dimension.

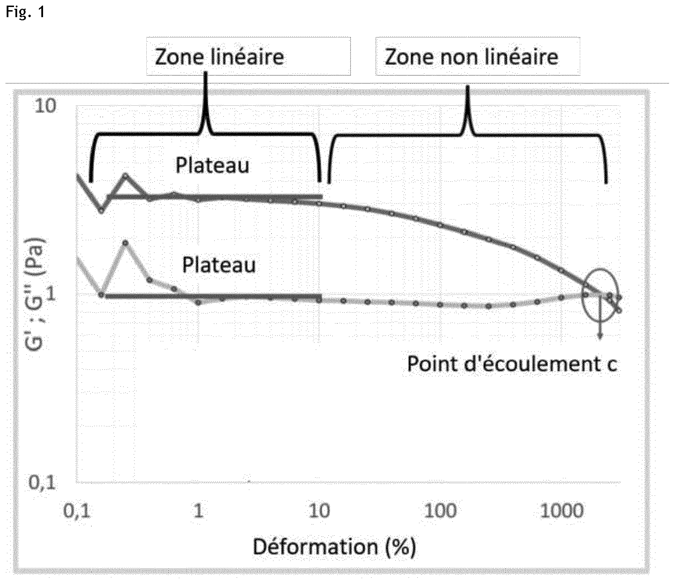

Par « **courbe de déformation** », on entend une courbe qui montre la relation entre l'élasticité (représentée par le module d'élasticité (G')) et/ou la viscosité (représentée par le module de viscosité (G")), par exemple en unité de Pascal (Pa), et la déformation (correspondant à la déformation relative, par exemple en pourcentage), pour un matériau donné (par exemple un échantillon de mucus). Une courbe de déformation comprend généralement une zone linéaire et une zone non linéaire. La zone linéaire comprend généralement une zone plateau (« p »). Un exemple de courbe de déformation représentant les modules G' et G", typiquement obtenue pour un échantillon de mucus, est donné par la figure 1.

Par « **zone linéaire d'une courbe de déformation** » ou **« zone linéaire** », « **plateau d'une courbe de déformation** » ou « **plateau** », on entend la portion de la courbe de déformation d'un matériau donné (par exemple un échantillon de mucus) où la contrainte est proportionnelle à la déformation et les modules G' et G" sont constantes. Dans le cas de la courbe de déformation d'un échantillon de mucus, la zone linéaire se situe généralement à une déformation inférieure ou égale à 10% (voir l'exemple représentatif donné à la figure 1), mais peut également se situer jusqu'à une déformation inférieure ou égale à 5% ou encore jusqu'à une déformation inférieure ou égale à 1%, selon l'échantillon.

Par « **zone non linéaire d'une courbe de déformation »** ou « **zone non linéaire** », on entend la portion de la courbe de déformation d'un matériau donné (par exemple un échantillon de mucus) où la contrainte est une fonction variable, généralement non linéaire, de la déformation. Dans le cas de la courbe de déformation d'un échantillon de mucus, la zone non linéaire commence généralement à une déformation supérieure à 10% (voir l'exemple représentatif donné à la figure 1), mais peut également commencer dès que la déformation atteint 5% ou encore dès que la déformation atteint 1%, selon l'échantillon.

Par « **point d'écoulement c d'une courbe de déformation** » ou « **point d'écoulement** » ou « **c** », on entend la déformation à partir de laquelle un matériau donné (par exemple un échantillon de mucus) entre en écoulement. Le point d'écoulement correspond au point de croisement de la courbe de déformation représentant le module d'élasticité (G') avec la courbe de déformation représentant le module de viscosité (G"), obtenues pour un matériau donné (voir l'exemple représentatif donné à la figure 1). En général, le point d'écoulement « c » d'un échantillon de mucus est atteint lorsque l'échantillon de mucus est soumis à une déformation allant de 400% à 10000%, de préférence de 500% à 9000%, de préférence de 600% à 8000%, de préférence de 700% à 7000%, de préférence de 800% à 6000%, de préférence de 900% à 5000%, de préférence de 1000% à 4000%, de préférence encore, à une déformation allant de 500% à 1000%.

Par « **mesure** » ou « **mesure d'une valeur** » ou « **mesure d'une grandeur** », on entend l'action d'évaluer une grandeur (appelée aussi valeur) d'après son rapport avec une grandeur de même espèce, prise comme unité et comme référence, en utilisant un appareil de mesure. Par « **détermination** » ou « **détermination d'une valeur** » ou « **détermination d'une grandeur** », on entend l'action dévaluer ou de calculer une grandeur (appelée aussi valeur) à partir d'une valeur ou d'une grandeur mesurée à l'aide d'un appareil de mesure.

Par « **module d'élasticité G'** » ou « **module d'élasticité** » ou « **module élastique** » ou « **G'** », on entend une grandeur intrinsèque d'un matériau, définie par le rapport d'une contrainte à la déformation élastique provoquée par cette contrainte. Les déformations étant sans dimension, les modules d'élasticité sont homogènes à une pression et leur unité SI est le pascal (Pa).

Par « **module de viscosité G"** » ou « **module de viscosité** » ou « **module visqueux »** ou « **G"** », on entend une grandeur intrinsèque d'un matériau qui caractérise son comportement visqueux (comprenant notamment l'énergie dissipée, par exemple sous forme de chaleur). L'unité SI du module de viscosité est le pascal (Pa).

Par « **module complexe G*** » ou « **module complexe** » ou « **G*** », on entend une grandeur représentative de la consistance générale du matériau, dont la valeur est égale à la racine carrée de la somme G'²+G"². L'unité SI du module complexe est le pascal (Pa).

Par « **facteur d'amortissement tan δ** », ou « **facteur d'amortissement** » ou « **tan δ** », on entend le facteur de perte. Le facteur d'amortissement correspond au rapport G"/G' et indique la pondération entre la réponse visqueuse et la réponse élastique du matériau sous une sollicitation mécanique donnée. Le facteur d'amortissement est une grandeur adimensionnelle.

Par « **déformation critique γ_{c} »** ou « **seuil de déformation à l'écoulement γ_{c}»** ou « **déformation critique** » ou « **seuil de déformation à l'écoulement** » ou « γ_{c} », on entend la déformation relative d'un matériau lorsqu'il entre en écoulement. La déformation critique est une grandeur adimensionnelle. On peut l'exprimer en pourcentage. Par « **déformation plastique** » ou « **déformation plastique γ_{P}** » ou **« déformation γ_{P}** » ou « **déformation au plateau** » ou « **γ_{P}** », on entend la valeur de la contrainte en fin de la zone de plateau de la courbe de déformation.

Par « **contrainte** τ » ou « **contrainte** » ou « **τ** », on entend une grandeur qui caractérise la force mécanique appliquée de manière tangentielle (parallèle) et/ou normale (perpendiculaire) à une face d'un matériau. La contrainte correspond au rapport d'une force à une surface. L'unité SI du seuil de contrainte est le pascal (Pa).

Par « **seuil de contrainte à l'écoulement** » ou « **seuil de contrainte à l'écoulement τ_{c}** » ou « τ_{c} » ou « **contrainte seuil** », on entend la valeur de la contrainte à exercer sur le matériau pour le mettre en écoulement. Il est généralement obtenu à partir d'une courbe d'écoulement. Par « **contrainte plastique** » ou « **contrainte plastique τₚ** » ou « **contrainte τₚ** » ou **« contrainte au plateau** » ou « τₚ », on entend la valeur de la contrainte en fin de la zone de plateau de la courbe de déformation.

Par « **force élastique EF** » ou « **force élastique** » ou « **EF** », on entend une grandeur dont la valeur est égale au produit du module d'élasticité G* mesuré dans la zone linéaire (plateau) de la courbe de déformation (paramètre appelé aussi G*ₚ) par la contrainte σ mesurée au point d'écoulement c de la courbe de déformation (paramètre appelé aussi σ_{c} ou contrainte critique), selon la formule EF=G*ₚσ_{c}. L'unité SI de la force élastique est le pascal au carré (Pa²).

Par « **contrainte critique σ_{c}»** ou « **contrainte critique** » ou « **σ_{c}** », on entend la contrainte minimale pour atteindre la zone d'écoulement, caractérisée par la rupture de la structure solide en fin de domaine plastique. A ce niveau de contrainte, la contribution visqueuse devient supérieure à la contribution élastique.

Par « **rhDNase** » ou « **désoxyribonucléase humaine recombinante** », on entend une enzyme d'origine humaine, catalysant l'hydrolyse (la coupure) des acides désoxyribonucléiques (ou ADN) en nucléotides ou polynucléotides. Elle hydrolyse les liaisons phosphodiesters. La rhDNase est notamment utilisée comme médicament ayant une action de fluidifiant bronchique chez des patients souffrant de maladies respiratoires et/ou pulmonaires. La rhDNase est généralement administrée au patient sous forme d'aérosols, par nébulisation, dans le but de traiter l'encombrement bronchique. Le traitement par la rhDNase vise à améliorer la fonction respiratoire des patients et à réduire le nombre d'exacerbations. Le traitement à la rhDNase modifie la rhéologie du mucus via une action sur l'ADN contenu dans les sécrétions. Les médicaments à base de rhDNase sont connus et disponibles dans le commerce (ils comprennent par exemple le Pulmozyme^{®} (dornase alfa ou rhDNase, Roche).

Par « **valeur limite »** ou « **valeur seuil** » ou « **valeur de référence** », on entend la valeur d'un paramètre rhéologique indiquant l'état d'un sujet au regard d'une maladie respiratoire et/ou pulmonaire. Selon le contexte, cette valeur correspond à la valeur d'un paramètre rhéologique déterminée ou mesurée sur un échantillon de mucus d'un sujet sain de référence ; ou à la moyenne des valeurs d'un paramètre rhéologique déterminées ou mesurées sur des échantillons de mucus différents d'un même sujet sain de référence (valeurs déterminées/mesurées sur des échantillons de mucus prélevés à des intervalles de temps séparés sur le même sujet de référence ou valeurs déterminées/mesurées sur le même échantillon de mucus à des intervalles de temps séparés) ou à la moyenne des valeurs d'un paramètre rhéologique déterminées/mesurées sur le mucus de plusieurs sujets sains de référence (au moins deux sujets sains de référence ) ; ou à la valeur d'un paramètre rhéologique déterminé/mesuré sur un échantillon de mucus d'un sujet de référence à un stade connu de la maladie des poumons et/ou de la maladie respiratoire ; ou à la moyenne des valeurs d'un paramètre rhéologique déterminées/mesurées sur des échantillons de mucus différents d'un même sujet de référence à un stade connu de la maladie des poumons et/ou de la maladie respiratoire (valeurs déterminées/mesurées sur des échantillons de mucus prélevés à des intervalles de temps séparés sur le même sujet de référence ou valeurs déterminées/mesurées sur le même échantillon de mucus à des intervalles de temps séparés) ou à la moyenne des valeurs d'un paramètre rhéologique déterminées/mesurées sur le mucus de plusieurs sujets de référence à un stade connu de la maladie des poumons et/ou de la maladie respiratoire (au moins deux sujets de référence à un stade connu de la maladie des poumons et/ou de la maladie respiratoire).

### Méthodes de diagnostic, de stratification, de pronostic, de suivi, d'évaluation de l'efficacité d'un traitement

Trois études cliniques menées par les Inventeurs ont permis de démontrer pour la première fois que les paramètres rhéologiques du mucus de patients atteints de différentes maladies respiratoires sont statistiquement différents d'une maladie à l'autre. Les Inventeurs ont notamment montré que ces paramètres rhéologiques peuvent être utilisés comme des biomarqueurs permettant de différencier les maladies respiratoires à la fois lorsqu'ils sont mesurés dans la zone linéaire de la courbe de déformation du mucus et à la fois lorsqu'ils sont mesurés dans la zone non linéaire de cette courbe. Les données montrent que la combinaison de la mesure d'un paramètre rhéologique dans la zone linéaire avec la mesure d'un paramètre rhéologique (identique ou différent) dans la zone linéaire de la courbe de déformation du mucus permet une discrimination particulièrement précise, efficace et significative de ces maladies. Les Inventeurs ont donc ainsi montré qu'il est possible de diagnostiquer, de stratifier, de pronostiquer ou de suivre de manière fiable et précise une maladie des poumons et/ou une maladie respiratoire chez un sujet, sur la base de la mesure de ces paramètres. Il est également possible d'évaluer l'efficacité d'un traitement administré à un sujet souffrant ou susceptible de souffrir d'une telle maladie. Les Inventeurs ont ainsi mis au point une nouvelle méthode permettant un diagnostic, une stratification, un pronostic et un suivi fiable des maladies des poumons et des maladies respiratoires, basée sur la mesure de paramètres rhéologiques dans la zone linéaire de la courbe de déformation et de paramètres rhéologiques dans la zone non linéaire de cette courbe obtenue à partir du mucus de patients.

Il est donc ici décrit donc une méthode *in vitro* de diagnostic, de stratification, de pronostic et/ou de suivi d'une maladie des poumons et/ou une maladie respiratoire, qui ne figure pas dans le texte des revendications, ladite méthode comprenant :
(i) la détermination ou la mesure de la valeur Aₙₗ d'au moins un paramètre rhéologique sur un échantillon de mucus A d'un sujet, dans une zone non linéaire de la courbe de déformation de l'échantillon ; et/ou
(ii) la détermination ou la mesure de la valeur Aₗ d'au moins un paramètre rhéologique sur ledit échantillon de mucus A, dans une zone linéaire de la courbe de déformation de l'échantillon ;

dans laquelle le paramètre rhéologique est choisi parmi :
   - le module d'élasticité (G'),
   - le module de viscosité (G"),
   - le facteur d'amortissement (tan δ),
   - le module complexe (G*),
   - la déformation plastique (γₚ),
   - la déformation critique (γ_{c}),
   - la contrainte plastique (τₚ),
   - le seuil de contrainte à l'écoulement (τ_{c}),
   - la force élastique (EF),
   - la contrainte critique (σ_{c}), et
   - une combinaison quelconque de ceux-ci ;
le paramètre rhéologique déterminé ou mesuré dans la zone non linéaire étant de préférence choisi parmi G', G", G*, tan δ, γ_{c}, τ_{c}, EF, σ_{c}, et une combinaison quelconque de ceux-ci ; de préférence parmi G*, tan δ, τ_{c}, EF, σ_{c}, et une combinaison quelconque de ceux-ci ; de préférence encore parmi τ_{c}, EF, σ_{c}, et une combinaison quelconque de ceux-ci, de préférence encore parmi τ_{c}, σ_{c}, et une combinaison quelconque de ceux-ci, de préférence encore, la détermination de la valeur Aₙₗ comprend au moins la détermination de la valeur du paramètre rhéologique σ_{c} ;
le paramètre rhéologique déterminé ou mesuré dans la zone linéaire étant de préférence choisi parmi G', G", G*, tan δ, γₚ, τₚ et une combinaison quelconque de ceux-ci ; de préférence parmi G*, tan δ, γₚ et une combinaison quelconque de ceux-ci ; de préférence encore parmi G*, tan δ et une combinaison quelconque de ceux-ci, de préférence encore, la détermination de la valeur Aₗ comprend au moins la détermination de la valeur du paramètre rhéologique G*.

La présente invention concerne donc une méthode *in vitro* de diagnostic, de stratification, de pronostic et/ou de suivi d'une maladie des poumons et/ou une maladie respiratoire, comprenant :
(i) la détermination de la valeur Aₙₗ d'au moins un paramètre rhéologique sur un échantillon de mucus A d'un sujet, dans une zone non linéaire de la courbe de déformation de l'échantillon ;
(ii) la détermination de la valeur Aₗ d'au moins un paramètre rhéologique sur ledit échantillon de mucus A, dans une zone linéaire de la courbe de déformation de l'échantillon ; et
(iii) le diagnostic, la stratification, le pronostic et/ou le suivi de la maladie des poumons et/ou de la maladie respiratoire à partir d'une comparaison desdites valeurs Aₙₗ, Aₗ déterminées avec une valeur limite respective ;

dans laquelle le paramètre rhéologique déterminé dans la zone non linéaire est choisi parmi :
   - le module d'élasticité (G'),
   - le module de viscosité (G"),
   - le facteur d'amortissement (tan δ),
   - le module complexe (G*),
   - la déformation critique (γ_{c}),
   - le seuil de contrainte à l'écoulement (τ_{c}),
   - la force élastique (EF),
   - la contrainte critique (σ_{c}), et
   - une combinaison quelconque de ceux-ci ;
le paramètre rhéologique déterminé dans la zone non linéaire comprenant au moins σ_{c}; et dans laquelle le paramètre rhéologique déterminé dans la zone linéaire est choisi parmi :
   - le module d'élasticité (G'),
   - le module de viscosité (G"),
   - le facteur d'amortissement (tan δ),
   - le module complexe (G*),
   - la déformation plastique (γₚ),
   - la contrainte plastique(τₚ), et
   - une combinaison quelconque de ceux-ci ;
le paramètre rhéologique déterminé dans la zone linéaire comprenant au moins G*.

La méthode *in vitro* de diagnostic, de stratification, de pronostic et/ou de suivi d'une maladie des poumons et/ou une maladie respiratoire, comprend au moins la détermination ou la mesure des paramètres rhéologiques σ_{c} et G*. Ainsi, la détermination ou la mesure de la valeur Aₙₗ d'au moins un paramètre rhéologique sur un échantillon de mucus A d'un sujet, dans une zone non linéaire de la courbe de déformation de l'échantillon, comprend au moins la détermination ou la mesure de la valeur Aₙₗ du paramètre rhéologique σ_{c}. La détermination ou la mesure de la valeur Aₗ d'au moins un paramètre rhéologique sur un échantillon de mucus A d'un sujet, dans une zone linéaire de la courbe de déformation de l'échantillon, comprend au moins la détermination ou la mesure de la valeur Aₗ du paramètre rhéologique G*. De préférence, le paramètre rhéologique déterminé ou mesuré dans la zone non linéaire consiste essentiellement en, ou est, le paramètre σ_{c}. De préférence, le paramètre rhéologique déterminé ou mesuré dans la zone consiste essentiellement en, ou est, G*. En effet, les Inventeurs ont montré que le diagnostic, la stratification, le pronostic et/ou le suivi d'une maladie des poumons et/ou une maladie respiratoire est(sont) particulièrement précis et déterminant(s) lorsque le paramètre déterminé ou mesuré dans la zone non linéaire est le paramètre σ_{c}. De la même façon, les Inventeurs ont montré que le diagnostic, la stratification, le pronostic et/ou le suivi d'une maladie des poumons et/ou une maladie respiratoire est(sont) particulièrement précis et déterminant(s) lorsque le paramètre déterminé ou mesuré dans la zone linéaire est paramètre G*.

La méthode *in vitro* de diagnostic, de stratification, de pronostic et/ou de suivi d'une maladie des poumons et/ou une maladie respiratoire, comprend au moins la détermination ou la mesure de la valeur Aₙₗ d'un paramètre rhéologique dans une zone non linéaire de la courbe de déformation de l'échantillon.

La méthode *in vitro* de diagnostic, de stratification, de pronostic et/ou de suivi d'une maladie des poumons et/ou une maladie respiratoire, comprend en outre au moins la détermination ou la mesure de la valeur Aₙₗ d'un paramètre rhéologique dans une zone non linéaire de la courbe de déformation de l'échantillon et au moins la détermination ou la mesure de la valeur Aₗ d'un paramètre rhéologique dans une zone linéaire de la courbe de déformation de l'échantillon, lesdits paramètres étant tels que définis ci-dessus. En effet, les Inventeurs ont montré que le diagnostic, la stratification, le pronostic et/ou le suivi d'une maladie des poumons et/ou une maladie respiratoire est(sont) particulièrement précis et déterminant(s) lorsque l'on détermine à la fois un paramètre rhéologique dans la zone non linéaire et un paramètre rhéologique dans la zone non linéaire de la courbe de déformation.

Ainsi, il est ici décrit une méthode *in vitro* de diagnostic, de stratification, de pronostic et/ou de suivi d'une maladie des poumons et/ou une maladie respiratoire, qui ne figure pas dans le texte des revendications, ladite méthode comprenant :
(i) la détermination ou la mesure de la valeur Aₙₗ d'au moins un paramètre rhéologique sur un échantillon de mucus A d'un sujet, dans une zone non linéaire de la courbe de déformation de l'échantillon ; et
(ii) la détermination ou la mesure de la valeur Aₗ d'au moins un paramètre rhéologique sur ledit échantillon de mucus A, dans une zone linéaire de la courbe de déformation de l'échantillon ;

dans laquelle le paramètre rhéologique est choisi parmi : le module d'élasticité (G'), le module de viscosité (G"), le facteur d'amortissement (tan δ), le module complexe (G*), la déformation plastique (γₚ), la déformation critique (γ_{c}), la contrainte plastique (τₚ), le seuil de contrainte à l'écoulement (τ_{c}), la force élastique (EF), la contrainte critique (σ_{c}), et une combinaison quelconque de ceux-ci ;
le paramètre rhéologique déterminé ou mesuré dans la zone non linéaire étant de préférence choisi parmi G', G", G*, tan δ, γ_{c}, τ_{c}, EF, σ_{c}, et une combinaison quelconque de ceux-ci ; de préférence parmi G*, tan δ, τ_{c}, EF, σ_{c}, et une combinaison quelconque de ceux-ci ; de préférence encore parmi τ_{c}, EF, σ_{c}, et une combinaison quelconque de ceux-ci ; de préférence encore parmi τ_{c}, σ_{c}, et une combinaison quelconque de ceux-ci ;
le paramètre rhéologique déterminé ou mesuré dans la zone non linéaire comprenant au moins σ_{c} ;
le paramètre rhéologique déterminé ou mesuré dans la zone linéaire étant de préférence choisi parmi G', G", G*, tan δ, γₚ, τₚ et une combinaison quelconque de ceux-ci ; de préférence parmi G*, tan δ, γₚ et une combinaison quelconque de ceux-ci ; de préférence encore parmi G*, tan δ et une combinaison quelconque de ceux-ci ;
le paramètre rhéologique déterminé ou mesuré dans la zone linéaire comprenant au moins G*.

Avantageusement, la méthode selon l'invention est une méthode *in vitro* de diagnostic et de stratification, ou de diagnostic et de pronostic, ou de diagnostic et de suivi, ou encore de stratification et de pronostic, ou de stratification et de suivi, ou encore de pronostic et de suivi, ou encore de diagnostic et de stratification et de pronostic, ou de de diagnostic et de stratification et de suivi, ou de diagnostic et de pronostic et de suivi, ou encore de stratification et de pronostic et de suivi, ou encore de diagnostic et de stratification et de pronostic et de suivi, d'une maladie des poumons et/ou une maladie respiratoire.

Il est en outre ici décrit une méthode *in vitro* d'évaluation de l'efficacité d'un traitement d'une maladie des poumons et/ou d'une maladie respiratoire, qui ne figure pas dans le texte des revendications, ladite méthode comprenant :
(i) la détermination ou la mesure de la valeur Aₙₗ d'au moins un paramètre rhéologique sur un échantillon de mucus A d'un sujet souffrant de la maladie des poumons et/ou de la maladie respiratoire et ayant reçu au moins une administration dudit traitement, dans une zone non linéaire de la courbe de déformation de l'échantillon ; et/ou
(ii) la détermination ou la mesure de la valeur Aₗ d'au moins un paramètre rhéologique sur ledit échantillon de mucus A, dans une zone linéaire de la courbe de déformation de l'échantillon ;

dans laquelle le paramètre rhéologique linéaire est choisi parmi :
   - le module d'élasticité (G'),
   - le module de viscosité (G"),
   - le facteur d'amortissement (tan δ),
   - le module complexe (G*),
   - la déformation plastique (γₚ),
   - la déformation critique (γ_{c}),
   - la contrainte plastique (τₚ),
   - le seuil de contrainte à l'écoulement (τ_{c}),
   - la force élastique (EF),
   - la contrainte critique (σ_{c}), et
   - une combinaison quelconque de ceux-ci ;
le paramètre rhéologique déterminé ou mesuré dans la zone non linéaire étant de préférence choisi parmi G', G", G*, tan δ, γ_{c}, τ_{c}, EF, σ_{c}, et une combinaison quelconque de ceux-ci ; de préférence parmi G*, tan δ, τ_{c}, EF, σ_{c}, et une combinaison quelconque de ceux-ci ; de préférence encore parmi τ_{c}, EF, σ_{c}, et une combinaison quelconque de ceux-ci ; de préférence encore parmi τ_{c}, σ_{c}, et une combinaison quelconque de ceux-ci ; de préférence encore, la détermination de la valeur Aₙₗ comprend au moins la détermination de la valeur du paramètre rhéologique σ_{c} ;
le paramètre rhéologique déterminé ou mesuré dans la zone linéaire étant de préférence choisi parmi G', G", G*, tan δ, γₚ, τₚ et une combinaison quelconque de ceux-ci; de préférence parmi G*, tan δ, γₚ et une combinaison quelconque de ceux-ci ; de préférence encore parmi G*, tan δ et une combinaison quelconque de ceux-ci ; de préférence encore, la détermination de la valeur Aₗ comprend au moins la détermination de la valeur du paramètre rhéologique G*.

L'invention concerne une méthode *in vitro* d'évaluation de l'efficacité d'un traitement d'une maladie des poumons et/ou d'une maladie respiratoire, comprenant :
(i) la détermination de la valeur Aₙₗ d'au moins un paramètre rhéologique sur un échantillon de mucus A d'un sujet souffrant de la maladie des poumons et/ou de la maladie respiratoire et ayant reçu au moins une administration dudit traitement, dans une zone non linéaire de la courbe de déformation de l'échantillon ; et
(ii) la détermination de la valeur Aₗ d'au moins un paramètre rhéologique sur ledit échantillon de mucus A, dans une zone linéaire de la courbe de déformation de l'échantillon ; et
(iii) l'évaluation de l'efficacité du traitement de la maladie des poumons et/ou de la maladie respiratoire à partir d'une comparaison desdites valeurs Aₙₗ, Aₗ déterminées avec une valeur limite respective ;

dans laquelle le paramètre rhéologique déterminé dans la zone non linéaire est choisi parmi :
   - le module d'élasticité (G'),
   - le module de viscosité (G"),
   - le facteur d'amortissement (tan δ),
   - le module complexe (G*),
   - la déformation critique (γ_{c}),
   - le seuil de contrainte à l'écoulement (τ_{c}),
   - la force élastique (EF),
   - la contrainte critique (σ_{c}), et
   - une combinaison quelconque de ceux-ci ;
le paramètre rhéologique déterminé dans la zone non linéaire comprenant au moins σ_{c}; et
dans laquelle le paramètre rhéologique déterminé dans la zone linéaire est choisi parmi :
   - le module d'élasticité (G'),
   - le module de viscosité (G"),
   - le facteur d'amortissement (tan δ),
   - le module complexe (G*),
   - la déformation plastique (γₚ),
   - la contrainte plastique(τₚ), et
   - une combinaison quelconque de ceux-ci ;
le paramètre rhéologique déterminé dans la zone linéaire comprenant au moins G*.

La méthode *in vitro* d'évaluation de l'efficacité d'un traitement d'une maladie des poumons et/ou d'une maladie respiratoire, comprend au moins la détermination ou la mesure des paramètres rhéologiques σ_{c} et G*. La détermination ou la mesure de la valeur Aₙₗ d'au moins un paramètre rhéologique sur un échantillon de mucus A d'un sujet, dans une zone non linéaire de la courbe de déformation de l'échantillon, comprend au moins la détermination ou la mesure de la valeur Aₙₗ du paramètre rhéologique σ_{c}. La détermination ou la mesure de la valeur Aₗ d'au moins un paramètre rhéologique sur un échantillon de mucus A d'un sujet, dans une zone linéaire de la courbe de déformation de l'échantillon, comprend au moins la détermination ou la mesure de la valeur Aₗ du paramètre rhéologique G*. De préférence, le paramètre rhéologique déterminé ou mesuré dans la zone non linéaire consiste essentiellement en, ou est, le paramètre σ_{c}. De préférence, le paramètre rhéologique déterminé ou mesuré dans la zone consiste essentiellement en, ou est, G*. En effet, les Inventeurs ont montré que l'évaluation de l'efficacité d'un traitement d'une maladie des poumons et/ou d'une maladie respiratoire est particulièrement précise et déterminante lorsque le paramètre déterminé ou mesuré dans la zone non linéaire est le paramètre σ_{c}. De la même façon, les Inventeurs ont montré que l'évaluation de l'efficacité d'un traitement d'une maladie des poumons et/ou d'une maladie respiratoire est particulièrement précise et déterminante lorsque le paramètre déterminé ou mesuré dans la zone linéaire est paramètre G*.

La méthode *in vitro* d'évaluation de l'efficacité d'un traitement d'une maladie des poumons et/ou d'une maladie respiratoire, comprend au moins la détermination ou la mesure de la valeur Aₙₗ d'un paramètre rhéologique dans une zone non linéaire de la courbe de déformation de l'échantillon.

Ainsi, la méthode *in vitro* d'évaluation de l'efficacité d'un traitement d'une maladie des poumons et/ou d'une maladie respiratoire, comprend au moins la détermination ou la mesure de la valeur Aₙₗ d'un paramètre rhéologique dans une zone non linéaire de la courbe de déformation de l'échantillon et au moins la détermination ou la mesure de la valeur Aₗ d'un paramètre rhéologique dans une zone linéaire de la courbe de déformation de l'échantillon, lesdits paramètres étant tels que définis ci-dessus. En effet, les Inventeurs ont montré que l'évaluation de l'efficacité d'un traitement d'une maladie des poumons et/ou d'une maladie respiratoire est particulièrement précise et déterminante lorsque l'on détermine à la fois un paramètre rhéologique dans la zone non linéaire et un paramètre rhéologique dans la zone non linéaire de la courbe de déformation.

Ainsi, il est donc ici décrit une méthode *in vitro* d'évaluation de l'efficacité d'un traitement d'une maladie des poumons et/ou d'une maladie respiratoire, qui ne figure pas dans le texte des revendications, ladite méthode comprenant :
(i) la détermination ou la mesure de la valeur Aₙₗ d'au moins un paramètre rhéologique sur un échantillon de mucus A d'un sujet, dans une zone non linéaire de la courbe de déformation de l'échantillon ; et
(ii) la détermination ou la mesure de la valeur Aₗ d'au moins un paramètre rhéologique sur ledit échantillon de mucus A, dans une zone linéaire de la courbe de déformation de l'échantillon ;

dans laquelle le paramètre rhéologique est choisi parmi : le module d'élasticité (G'), le module de viscosité (G"), le facteur d'amortissement (tan δ), le module complexe (G*), la déformation plastique (γₚ), la déformation critique (γ_{c}), la contrainte plastique (τₚ), le seuil de contrainte à l'écoulement (τ_{c}), la force élastique (EF), la contrainte critique (σ_{c}), et une combinaison quelconque de ceux-ci ;
le paramètre rhéologique déterminé ou mesuré dans la zone non linéaire étant de préférence choisi parmi G', G", G*, tan δ, γ_{c}, τ_{c}, EF, σ_{c}, et une combinaison quelconque de ceux-ci ; de préférence parmi G*, tan δ, τ_{c}, EF, σ_{c}, et une combinaison quelconque de ceux-ci ; de préférence encore parmi τ_{c}, EF, σ_{c}, et une combinaison quelconque de ceux-ci ; de préférence encore parmi τ_{c}, σ_{c}, et une combinaison quelconque de ceux-ci ;
le paramètre rhéologique déterminé ou mesuré dans la zone non linéaire comprenant au moins σ_{c} ;
le paramètre rhéologique déterminé ou mesuré dans la zone linéaire étant de préférence choisi parmi G', G", G*, tan δ, γₚ, τₚ et une combinaison quelconque de ceux-ci; de préférence parmi G*, tan δ, γₚ et une combinaison quelconque de ceux-ci ; de préférence encore parmi G*, tan δ et une combinaison quelconque de ceux-ci ;
le paramètre rhéologique déterminé ou mesuré dans la zone linéaire comprenant au moins G*.

L'échantillon de mucus peut avoir été prélevé par une expectoration spontanée d'un sujet. L'échantillon de mucus peut avoir également été obtenu par un prélèvement selon différentes techniques, incluant par exemple le lavage bronchiolo-alvéolaire (LBA), l'écouvillonnage pharyngé, l'aspiration pharyngée et l'expectoration induite.

Le lavage bronchiolo-alvéolaire est une technique de prélèvement au cours d'une fibroscopie bronchique. Le fibroscope est bloqué dans une bronche périphérique et du sérum physiologique est injecté puis réaspiré. Des précautions doivent être prises pour éviter la contamination du prélèvement par les sécrétions nasopharyngées. L'écouvillonnage pharyngé consiste à recueillir des sécrétions sur un écouvillon, soit par frottement de la paroi postérieure du pharynx soit en demandant au patient de tousser sur l'écouvillon. L'aspiration pharyngée consiste à aspirer le contenu du pharynx postérieur grâce à un petit cathéter, soit par voie buccale en utilisant un abaisse-langue, soit par voie nasale. L'expectoration induite consiste à favoriser l'expectoration par l'inhalation de sérum salé hypertonique (comprenant du NaCl ou autre), qui permet d'augmenter le volume de sécrétions et d'améliorer la clearance muco-ciliaire des voies aériennes inférieures chez des sujets qui ne crachent pas. L'expectoration peut notamment être induite par inhalation de concentrations croissantes (3 % à 5 %) de sérum salé hypertonique par périodes de 5 à 7 minutes, par exemple à l'aide d'un nébuliseur ultrasonique.

Le sujet peut être un sujet sain. Le sujet peut également être un sujet susceptible d'être atteint d'une maladie pulmonaire et/ou respiratoire. Cela peut être par exemple le cas lorsque le sujet présente des symptômes d'une telle maladie, ou en cas de prédisposition à une telle maladie. La prédisposition peut être génétique et/ou héréditaire. Lorsque l'invention concerne une méthode *in vitro* de diagnostic, d'une maladie des poumons et/ou une maladie respiratoire, le sujet est de préférence un sujet susceptible d'être atteint d'une maladie pulmonaire et/ou respiratoire.

Alternativement, le sujet peut être un sujet souffrant d'une maladie pulmonaire et/ou respiratoire, c'est-à-dire un sujet dont ladite maladie est déclarée et a été diagnostiquée au préalable. Lorsque l'invention concerne une méthode *in vitro* de stratification, de pronostic et/ou de suivi d'une maladie des poumons et/ou une maladie respiratoire, le sujet est de préférence un sujet souffrant d'une telle maladie.

Le sujet peut être sous traitement d'une maladie pulmonaire et/ou respiratoire. C'est notamment le cas lorsque l'invention concerne une méthode *in vitro* d'évaluation de l'efficacité d'un traitement d'une maladie des poumons et/ou d'une maladie respiratoire. Cela peut être également le cas lorsque l'invention concerne une méthode *in vitro* de diagnostic, de stratification, de pronostic et/ou de suivi d'une maladie des poumons et/ou une maladie respiratoire. Le traitement peut être préventif, par exemple en cas de prédisposition à une maladie, ou il peut être curatif, par exemple en cas de maladie diagnostiquée/déclarée. Dans ce cas, le sujet a reçu au moins une administration dudit traitement. Le traitement peut être un traitement connu de l'homme du métier, administré selon un protocole classiquement suivi par l'homme du métier pour traiter la maladie pulmonaire et/ou respiratoire dont souffre le patient. Ils comprennent notamment les bronchodilatateurs, les corticostéroïdes, les antibiotiques, l'oxygénothérapie, les vaccins, les anticorps monoclonaux (tel que le Dupilumab), les correcteurs de CFTR (pour Cystic fibrosis transmembrane conductance ; traitements ciblant la protéine CFTR), les correcteurs du canal sodique épithélial (ENaC, pour Epithelial Na channel), et les combinaisons de ceux-ci.

Le traitement peut également être expérimental, c'est-à-dire un traitement dont les effets sur la maladie respiratoire et/ou pulmonaires considérée ne sont pas encore connus (ou mal connus). Dans ce cas, les méthodes selon l'invention permettent d'évaluer si ce traitement peut être utilisé pour traiter la maladie visée. Lorsque l'invention concerne une méthode *in vitro* de stratification, de pronostic et/ou de suivi d'une maladie des poumons et/ou une maladie respiratoire, le sujet traité est de préférence un sujet souffrant d'une telle maladie. L'homme du métier saura définir le ou les médicament(s) adapté(s) et/ou la ou les thérapie(s) adaptée(s) à administrer au sujet et/ou à expérimenter, ainsi que le protocole à mettre en oeuvre et les modalités de l'administration, selon le sujet considéré, la maladie diagnostiquée ou suspectée et/ou le stade diagnostiqué ou suspecté de la maladie.

Alternativement, le sujet peut n'être sous aucun traitement d'une maladie pulmonaire et/ou respiratoire. Par exemple, le sujet peut n'avoir reçu aucune administration d'un traitement donné, connu de l'homme du métier pour une maladie pulmonaire et/ou respiratoire, au cours d'une période déterminée précédent la mise en oeuvre d'une méthode selon l'invention, ladite période ayant une durée pouvant aller d'une à plusieurs semaines (par exemple 2 à 8 semaines) ou encore d'un à plusieurs mois (par exemple 3 à 10 mois) ou encore d'une à plusieurs années (par exemple 1 à 20 ans, ou encore 2 à 15 ans ou encore 3 à 12 ans ou encore 3 à 10 ans). Par exemple, le sujet peut n'avoir reçu aucune administration d'un traitement donné, connu de l'homme du métier pour une maladie pulmonaire et/ou respiratoire au cours de sa vie. L'homme du métier saura définir la durée de la période précédant la mise en oeuvre d'une méthode selon l'invention durant laquelle le sujet ne devra pas avoir reçu un traitement donné. Cette durée pourra par exemple dépendre de la pharmacocinétique du traitement, de sa clairance et ou de sa durée d'élimination de l'organisme.

Selon un mode de réalisation préféré des méthodes de l'invention, le sujet est un humain.

Afin de mesurer et/ou de déterminer le paramètre rhéologique, l'échantillon de mucus est soumis à une déformation ou à une contrainte en vue de générer une courbe du type de la figure 1. Les rhéomètres fonctionnent classiquement :
- soit en appliquant une déformation et en mesurant la contrainte (figure 1),
- soit en appliquant une contrainte et en mesurant la déformation (en pratique via une boucle de rétroaction).

Cette déformation est appliquée au moyen d'un rhéomètre, dans lequel l'échantillon est placé entre deux parties mobiles, par exemple en rotation ou en oscillation, l'une par rapport à l'autre. Une première partie est entraînée en rotation par rapport à la seconde partie de sorte à appliquer une contrainte de cisaillement à l'échantillon. Un capteur permet de mesurer le couple appliqué par l'échantillon à la seconde partie.

De manière particulièrement avantageuse, la surface de chaque partie en contact avec l'échantillon présente une rugosité suffisante pour minimiser voire éviter le glissement de l'échantillon le long de ladite surface.

Il existe différents types de rhéomètres, associés à des géométries différentes des deux parties (cylindres coaxiaux, cône-plan, plan-plan), mais l'invention n'est pas limitée à un type de rhéomètre particulier.

Les résultats présentés dans le présent texte ont été obtenus avec un rhéomètre de type plan-plan, dont les caractéristiques sont fournies plus bas dans la section « Matériel et méthodes ».

Selon un mode de réalisation préféré des méthodes de l'invention, la valeur Aₗ d'au moins un paramètre rhéologique est déterminée/mesurée à une déformation inférieure ou égale à 10%, de préférence à une déformation inférieure ou égale à 9%, de préférence à une déformation inférieure ou égale à 8%, de préférence à une déformation inférieure ou égale à 7%, de préférence à une déformation inférieure ou égale à 6%, de préférence à une déformation inférieure ou égale à 5%, de préférence à une déformation inférieure ou égale à 4%, de préférence à une déformation inférieure ou égale à 3%, de préférence à une déformation inférieure ou égale à 2%, de préférence à une déformation inférieure ou égale à 1%. Selon un mode de réalisation préféré des méthodes de l'invention, la valeur Aₗ d'au moins un paramètre rhéologique est déterminée/mesurée à une déformation allant de 0% à 10%, de préférence à une déformation allant de 0,5% à 10%, de préférence à une déformation allant de 1% à 9%, de préférence à une déformation allant de 2% à 8%, de préférence à une déformation allant de 3% à 7%, de préférence à une déformation allant de 4% à 6%, de préférence à une déformation d'environ 5%. Selon un mode de réalisation préféré des méthodes de l'invention, la valeur Aₗ d'au moins un paramètre rhéologique est déterminée/mesurée à une déformation d'environ 10%, de préférence d'environ 9%, de préférence d'environ 8%, de préférence d'environ 7%, de préférence d'environ 6%, de préférence d'environ 5%, de préférence d'environ 4%, de préférence d'environ 3%, de préférence d'environ 2%, de préférence d'environ 1%, de préférence d'environ 0%. Selon un mode de réalisation préféré des méthodes de l'invention, la valeur Aₗ d'au moins un paramètre rhéologique est déterminée/mesurée à une déformation égale à 10%, de préférence égale à 9%, de préférence égale à 8%, de préférence égale à 7%, de préférence égale à 6%, de préférence égale à 5%, de préférence égale à 4%, de préférence égale à 3%, de préférence égale à 2%, de préférence égale à 1%, de préférence égale à 0%. Selon un mode de réalisation particulièrement préféré, la valeur Aₗ d'au moins un paramètre rhéologique est déterminée/mesurée à une déformation égale à 5% ou d'environ 5%. Selon un mode de réalisation particulièrement préféré, lorsque le paramètre rhéologique est G', G", G* ou tan δ, la valeur Aₗ est déterminée/mesurée à une déformation égale à 5% ou d'environ 5%. En effet, les données cliniques montrent que les différentes maladies respiratoires et/ou pulmonaires sont discriminés de manière particulièrement significative lorsque la mesure des paramètres rhéologiques est effectuée à une déformation de 5%, notamment lorsque le paramètre est G', G", G* ou tan δ. En outre, les données montrent que la répétabilité est particulièrement bonne lorsque la mesure des paramètres rhéologiques est effectuée à une déformation de 5%.

Alternativement ou en combinaison, la valeur Aₙₗ d'au moins un paramètre rhéologique est de préférence déterminée/mesurée à une déformation supérieure à 10%, de préférence à une déformation supérieure ou égale à 15%, de préférence à une déformation supérieure ou égale à 20%, de préférence à une déformation supérieure ou égale à 30%, de préférence à une déformation supérieure ou égale à 40%, de préférence à une déformation supérieure ou égale à 50%, de préférence à une déformation supérieure ou égale à 60%, de préférence à une déformation supérieure ou égale à 70%, de préférence à une déformation supérieure ou égale à 80%, de préférence à une déformation supérieure ou égale à 90%, de préférence à une déformation supérieure ou égale à 100%, de préférence à une déformation supérieure ou égale à 150%, de préférence à une déformation supérieure ou égale à 200%, de préférence à une déformation supérieure ou égale à 300%, de préférence à une déformation supérieure ou égale à 400%, de préférence à une déformation supérieure ou égale à 500%, de préférence à une déformation supérieure ou égale à 1000%.

Les résultats obtenus par les Inventeurs montrent que les différentes maladies respiratoires et/ou pulmonaires sont discriminées de manière particulièrement précise et significative lorsque la mesure des paramètres rhéologiques est effectuée à une déformation correspondant au point d'écoulement « c » de l'échantillon de mucus, notamment lorsque le paramètre est G', G", G*, τ_{c}, σ_{c}, ou EF, en particulier lorsque le paramètre est σ_{c}. En outre, les données montrent que la répétabilité est particulièrement bonne lorsque la mesure des paramètres rhéologiques est effectuée au point d'écoulement « c » de l'échantillon de mucus. Ainsi, selon un mode de réalisation préféré des méthodes selon l'invention, la valeur Aₙₗ d'au moins un paramètre rhéologique est déterminée/mesurée au point d'écoulement « c » de l'échantillon de mucus. Selon un mode de réalisation particulièrement préféré, lorsque le paramètre rhéologique est G', G", G*, τ_{c}, σ_{c}, ou EF, la valeur Aₙₗ est déterminée/mesurée « au point d'écoulement « c » de l'échantillon de mucus.

La valeur Aₗ déterminée/mesurée est comparée à une valeur limite et la valeur Aₙₗ déterminée/mesurée est comparée à une valeur limite. Cette comparaison permet notamment de déterminer le diagnostic, la stratification, le pronostic et/ou le suivi d'une maladie des poumons et/ou une maladie respiratoire chez le sujet, et/ou de déterminer l'efficacité d'un traitement d'une maladie des poumons et/ou d'une maladie respiratoire chez un sujet ayant reçu au moins une administration dudit traitement.

Selon un mode de réalisation particulier des méthodes selon l'invention, le sujet est atteint d'une maladie des poumons et/ou d'une maladie respiratoire, ou le pronostic d'une maladie des poumons et/ou d'une maladie respiratoire est négatif, ou une maladie des poumons et/ou une maladie respiratoire est exacerbée, ou le traitement d'une maladie des poumons et/ou d'une maladie respiratoire est inefficace ou peu efficace, si au moins une des valeurs Aₗ et/ou Aₙₗ déterminée/mesurées est différente de la valeur limite respective.

Selon un mode de réalisation préféré, le sujet est atteint d'une maladie des poumons et/ou d'une maladie respiratoire, ou le pronostic d'une maladie des poumons et/ou d'une maladie respiratoire est négatif, ou une maladie des poumons et/ou une maladie respiratoire est exacerbée, ou le traitement d'une maladie des poumons et/ou d'une maladie respiratoire est inefficace ou peu efficace, si au moins une des valeurs Aₗ et/ou Aₙₗ déterminée/mesurées est supérieure à la valeur limite respective, lorsque :
- la valeur limite est la valeur d'un paramètre rhéologique déterminé/mesuré sur le mucus d'un sujet sain de référence ; ou la moyenne des valeurs d'un paramètre rhéologique déterminées/mesurées sur des échantillons de mucus différents d'un même sujet sain de référence (valeurs déterminées/mesurées sur des échantillons de mucus prélevés à des intervalles de temps séparés sur le même sujet de référence ou valeurs déterminées/mesurées sur le même échantillon de mucus à des intervalles de temps séparés) ; ou la moyenne des valeurs d'un paramètre rhéologique déterminé/mesuré sur le mucus de plusieurs sujets sains de référence (au moins deux sujets sains de référence) ; ou à la valeur d'un paramètre rhéologique déterminé/mesuré sur le mucus d'un sujet de référence à un stade léger ou modéré de la maladie des poumons et/ou de la maladie respiratoire ; ou la moyenne des valeurs d'un paramètre rhéologique déterminé/mesurées sur des échantillons de mucus différents d'un même sujet de référence à un stade léger ou modéré de la maladie des poumons et/ou de la maladie respiratoire (valeurs déterminées/mesurées sur des échantillons de mucus prélevés à des intervalles de temps séparés sur le même sujet de référence ou valeurs déterminées/mesurées sur le même échantillon de mucus à des intervalles de temps séparés) ; ou la moyenne des valeurs d'un paramètre rhéologique déterminé/mesuré sur le mucus de plusieurs sujets de référence à un stade léger ou modéré de la maladie des poumons et/ou de la maladie respiratoire (au moins deux sujets de référence à un stade connu de la maladie des poumons et/ou de la maladie respiratoire) ; et
- le paramètre rhéologique déterminé/mesuré dans la zone non linéaire est choisi parmi, et/ou comprend, ou consiste essentiellement en, ou consiste en, au moins un parmi, G', G", G*, γ_{c}, τ_{c} σ_{c}, EF, et une combinaison quelconque de ceux-ci , de préférence le paramètre rhéologique déterminé/mesuré dans la zone non linéaire est choisi parmi, et/ou comprend, ou consiste essentiellement en, ou consiste en, au moins un parmi, G', G", G*, σ_{c}, EF, et une combinaison quelconque de ceux-ci, de préférence encore le paramètre rhéologique déterminé/mesuré dans la zone non linéaire est choisi parmi, et/ou comprend, ou consiste essentiellement en, ou consiste en, au moins un parmi, G*, σ_{c}, et EF, de préférence encore parmi EF, σ_{c}, et une combinaison quelconque de ceux-ci ; de préférence encore, le paramètre rhéologique déterminé/mesuré dans la zone non linéaire comprend au moins σ_{c}, ou consiste essentiellement en σ_{c}, ou est σ_{c} ; et/ou
- le paramètre rhéologique déterminé/mesuré dans la zone linéaire est choisi parmi, et/ou comprend, ou consiste essentiellement en, ou consiste en, au moins un parmi, G', G", G*, γₚ, τₚ, et une combinaison quelconque de ceux-ci, de préférence le paramètre rhéologique mesuré dans la zone linéaire est choisi parmi, et/ou comprend, ou consiste essentiellement en, ou consiste en, au moins un parmi, G', G", G*, et une combinaison quelconque de ceux-ci; de préférence encore le paramètre rhéologique déterminé/mesuré dans la zone linéaire comprend au moins G*, ou consiste essentiellement en G*, ou est G*.

A l'inverse, selon un mode de réalisation préféré, le sujet n'est pas atteint d'une maladie des poumons et/ou d'une maladie respiratoire, ou le pronostic d'une maladie des poumons et/ou d'une maladie respiratoire est positif, ou une maladie des poumons et/ou une maladie respiratoire n'est pas exacerbée, ou le traitement d'une maladie des poumons et/ou d'une maladie respiratoire est efficace si au moins une des valeurs Aₗ et/ou Aₙₗ déterminées/mesurées est inférieure à la valeur limite respective, lorsque :
- la valeur limite est la valeur d'un paramètre rhéologique déterminé/mesuré sur le mucus d'un sujet sain ; ou la moyenne des valeurs d'un paramètre rhéologique déterminées/mesurées sur des échantillons de mucus différents d'un même sujet sain de référence (valeurs déterminées/mesurées sur des échantillons de mucus prélevés à des intervalles de temps séparés sur le même sujet de référence ou valeurs déterminées/mesurées sur le même échantillon de mucus à des intervalles de temps séparés) ; ou la moyenne des valeurs d'un paramètre rhéologique déterminé/mesuré sur le mucus de plusieurs sujets sains de référence (au moins deux sujets sains de référence) ; ou à la valeur d'un paramètre rhéologique déterminé/mesuré sur le mucus d'un sujet de référence à un stade léger ou modéré de la maladie des poumons et/ou de la maladie respiratoire ; ou la moyenne des valeurs d'un paramètre rhéologique déterminées/mesurées sur des échantillons de mucus différents d'un même sujet de référence à un stade léger ou modéré de la maladie des poumons et/ou de la maladie respiratoire (valeurs déterminées/mesurées sur des échantillons de mucus prélevés à des intervalles de temps séparés sur le même sujet de référence ou valeurs déterminées/mesurées sur le même échantillon de mucus à des intervalles de temps séparés) ; ou la moyenne des valeurs d'un paramètre rhéologique déterminé/mesuré sur le mucus de plusieurs sujets de référence à un stade léger ou modéré de la maladie des poumons et/ou de la maladie respiratoire (au moins deux sujets de référence à un stade connu de la maladie des poumons et/ou de la maladie respiratoire) ; et
- le paramètre rhéologique déterminé/mesuré dans la zone non linéaire est choisi parmi, et/ou comprend, ou consiste essentiellement en, ou consiste en, au moins un parmi, G', G", G*, γ_{c}, τ_{c}, σ_{c}, EF, et une combinaison quelconque de ceux-ci , de préférence le paramètre rhéologique déterminé/mesuré dans la zone non linéaire est choisi parmi, et/ou comprend, ou consiste essentiellement en, ou consiste en, au moins un parmi, G', G", G*, σ_{c}, EF, et une combinaison quelconque de ceux-ci, de préférence encore le paramètre rhéologique déterminé/mesuré dans la zone non linéaire est choisi parmi, et/ou comprend, ou consiste essentiellement en, ou consiste en, au moins un parmi, G*, σ_{c}, et EF, de préférence encore parmi EF, σ_{c}, et une combinaison quelconque de ceux-ci ; de préférence encore, le paramètre rhéologique déterminé/mesuré dans la zone non linéaire comprend au moins σ_{c}, ou consiste essentiellement en σ_{c}, ou est σ_{c}; et/ou
- le paramètre rhéologique déterminé/mesuré dans la zone linéaire est choisi parmi, et/ou comprend, ou consiste essentiellement en, ou consiste en, au moins un parmi, G', G", G*, γₚ, τₚ, et une combinaison quelconque de ceux-ci, de préférence le paramètre rhéologique mesuré dans la zone linéaire est choisi parmi, et/ou comprend, ou consiste essentiellement en, ou consiste en, au moins un parmi, G', G", G*, et une combinaison quelconque de ceux-ci; de préférence encore le paramètre rhéologique déterminé/mesuré dans la zone linéaire comprend au moins G*, ou consiste essentiellement en G*, ou est G*.

Selon un mode de réalisation préféré, le sujet est atteint d'une maladie des poumons et/ou d'une maladie respiratoire, ou le pronostic d'une maladie des poumons et/ou d'une maladie respiratoire est négatif, ou une maladie des poumons et/ou une maladie respiratoire est exacerbée, ou le traitement d'une maladie des poumons et/ou d'une maladie respiratoire est inefficace ou peu efficace, si au moins une des valeurs Aₗ et/ou Aₙₗ déterminées/mesurées est inférieure à la valeur limite respective, lorsque :
- la valeur limite est la valeur d'un paramètre rhéologique déterminé/mesuré sur le mucus d'un sujet sain ; ou la moyenne des valeurs d'un paramètre rhéologique déterminées/mesurées sur des échantillons de mucus différents d'un même sujet sain de référence (valeurs déterminées/mesurées sur des échantillons de mucus prélevés à des intervalles de temps séparés sur le même sujet de référence ou valeurs déterminées/mesurées sur le même échantillon de mucus à des intervalles de temps séparés) ; ou la moyenne des valeurs d'un paramètre rhéologique déterminé/mesuré sur le mucus de plusieurs sujets sains de référence (au moins deux sujets sains de référence ) ; ou à la valeur d'un paramètre rhéologique déterminé/mesuré sur le mucus d'un sujet de référence à un stade léger ou modéré de la maladie des poumons et/ou de la maladie respiratoire ; ou la moyenne des valeurs d'un paramètre rhéologique déterminées/mesurées sur des échantillons de mucus différents d'un même sujet de référence à un stade léger ou modéré de la maladie des poumons et/ou de la maladie respiratoire (valeurs déterminées/mesurées sur des échantillons de mucus prélevés à des intervalles de temps séparés sur le même sujet de référence ou valeurs déterminées/mesurées sur le même échantillon de mucus à des intervalles de temps séparés) ; ou la moyenne des valeurs d'un paramètre rhéologique déterminé/mesuré sur le mucus de plusieurs sujets de référence à un stade léger ou modéré de la maladie des poumons et/ou de la maladie respiratoire (au moins deux sujets de référence à un stade connu de la maladie des poumons et/ou de la maladie respiratoire) ; et
- le paramètre rhéologique déterminé/mesuré dans la zone non linéaire comprend au moins tan δ, ou consiste essentiellement en tan δ, ou est tan δ; et/ou
- le paramètre rhéologique déterminé/mesuré dans la zone linéaire comprend au moins tan δ, ou consiste essentiellement en tan δ, ou est tan δ.

A l'inverse, selon un mode de réalisation préféré, le sujet n'est pas atteint d'une maladie des poumons et/ou d'une maladie respiratoire, ou le pronostic d'une maladie des poumons et/ou d'une maladie respiratoire est positif, ou une maladie des poumons et/ou une maladie respiratoire n'est pas exacerbée, ou le traitement d'une maladie des poumons et/ou d'une maladie respiratoire est efficace si au moins une des valeurs Aₗ et/ou Aₙₗ déterminées/mesurées est supérieure à la valeur limite respective, lorsque :
- la valeur limite est la valeur d'un paramètre rhéologique déterminé/mesuré sur le mucus d'un sujet sain ; ou la moyenne des valeurs d'un paramètre rhéologique déterminées/mesurées sur des échantillons de mucus différents d'un même sujet sain de référence (valeurs déterminées/mesurées sur des échantillons de mucus prélevés à des intervalles de temps séparés sur le même sujet de référence ou valeurs déterminées/mesurées sur le même échantillon de mucus à des intervalles de temps séparés) ; ou la moyenne des valeurs d'un paramètre rhéologique déterminé/mesuré sur le mucus de plusieurs sujets sains de référence (au moins deux sujets sains de référence ) ; ou à la valeur d'un paramètre rhéologique déterminé/mesuré sur le mucus d'un sujet de référence à un stade léger ou modéré de la maladie des poumons et/ou de la maladie respiratoire ; ou la moyenne des valeurs d'un paramètre rhéologique déterminées/mesurées sur des échantillons de mucus différents d'un même sujet de référence à un stade léger ou modéré de la maladie des poumons et/ou de la maladie respiratoire (valeurs déterminées/mesurées sur des échantillons de mucus prélevés à des intervalles de temps séparés sur le même sujet de référence ou valeurs déterminées/mesurées sur le même échantillon de mucus à des intervalles de temps séparés) ; ou la moyenne des valeurs d'un paramètre rhéologique déterminé/mesuré sur le mucus de plusieurs sujets de référence à un stade léger ou modéré de la maladie des poumons et/ou de la maladie respiratoire (au moins deux sujets de référence à un stade connu de la maladie des poumons et/ou de la maladie respiratoire) ; et
- le paramètre rhéologique déterminé/mesuré dans la zone non linéaire comprend au moins tan δ, ou consiste essentiellement en tan δ, ou est tan δ; et/ou
- le paramètre rhéologique déterminé/mesuré dans la zone linéaire comprend au moins tan δ, ou consiste essentiellement en tan δ, ou est tan δ.

Selon un mode de réalisation particulièrement préféré, la valeur limite est la valeur d'un paramètre rhéologique déterminé/mesuré sur le mucus d'un sujet sain de référence ; ou la moyenne des valeurs d'un paramètre rhéologique déterminées/mesurées sur des échantillons de mucus différents d'un même sujet sain de référence (valeurs déterminées/mesurées sur des échantillons de mucus prélevés à des intervalles de temps séparés sur le même sujet de référence (i.e. sujet unique) ou valeurs déterminées/mesurées sur le même échantillon de mucus à des intervalles de temps séparés) ; ou la moyenne des valeurs d'un paramètre rhéologique déterminé/mesuré sur le mucus de plusieurs sujets sains de référence (au moins deux sujets sains de référence). De préférence, le sujet sain de référence ne souffre d'aucune maladie des poumons et/ou respiratoire.

Selon un mode de réalisation, lorsque la valeur limite d'un paramètre rhéologique est choisie parmi la valeur d'un paramètre rhéologique déterminé/mesuré sur le mucus d'un sujet de référence à un stade connu de la maladie des poumons et/ou de la maladie respiratoire ; ou à la moyenne des valeurs d'un paramètre rhéologique déterminées/mesurées sur des échantillons de mucus différents d'un sujet de référence à un stade connu de la maladie des poumons et/ou de la maladie respiratoire (valeurs déterminées/mesurées sur des échantillons de mucus prélevés à des intervalles de temps séparés sur le même sujet de référence ou valeurs déterminées/mesurées sur le même échantillon de mucus à des intervalles de temps séparés) ou à la moyenne des valeurs d'un paramètre rhéologique déterminé/mesuré sur le mucus de plusieurs sujets de référence à un stade connu de la maladie des poumons et/ou de la maladie respiratoire (au moins deux sujets de référence à un stade connu de la maladie des poumons et/ou de la maladie respiratoire), le sujet est :
- au même stade de la maladie que le ou les sujets de référence si au moins une des valeurs Aₗ et/ou Aₙₗ déterminées/mesurées est égale à la valeur limite du paramètre rhéologique, ou si au moins une des valeurs Aₗ et/ou Aₙₗ déterminées/mesurées ne diffère pas de plus de 100% de la valeur limite du paramètre rhéologique, de préférence ne diffère pas de plus de 90%, de préférence ne diffère pas de plus de 80%, de préférence ne diffère pas de plus de 70%, de préférence ne diffère pas de plus de 60%, de préférence ne diffère pas de plus de 50%, de préférence ne diffère pas de plus de 40%, de préférence ne diffère pas de plus de 30%, de préférence ne diffère pas de plus de 20%, de préférence ne diffère pas de plus de 10%, de préférence ne diffère pas de plus de 9%, de préférence ne diffère pas de plus de 8%, de préférence ne diffère pas de plus de 7%, de préférence ne diffère pas de plus de 6%, de préférence ne diffère pas de plus de 5%, de préférence ne diffère pas de plus de 4%, de préférence ne diffère pas de plus de 3%, de préférence ne diffère pas de plus de 2%, de préférence ne diffère pas de plus de 1% de la valeur limite du paramètre rhéologique ;
- à un stade supérieur (i.e. plus grave/avancé) de la maladie que le ou les sujets de référence si au moins une des valeurs Aₗ et/ou Aₙₗ déterminées/mesurées est supérieure d'au moins 100% de la valeur limite du paramètre rhéologique, de préférence d'au moins 150%, de préférence d'au moins 200%, de préférence d'au moins 300%, de préférence d'au moins 400%, lorsque :
   ∘ le paramètre rhéologique déterminé/mesuré dans la zone non linéaire est choisi parmi, et/ou comprend, ou consiste essentiellement en, ou consiste en, au moins un parmi, G', G", G*, γ_{c}, τ_{c}, σ_{c}, EF, et une combinaison quelconque de ceux-ci , de préférence le paramètre rhéologique déterminé/mesuré dans la zone non linéaire est choisi parmi, et/ou comprend, ou consiste essentiellement en, ou consiste en, au moins un parmi, G', G", G*, σ_{c}, EF, et une combinaison quelconque de ceux-ci, de préférence encore le paramètre rhéologique déterminé/mesuré dans la zone non linéaire est choisi parmi, et/ou comprend, ou consiste essentiellement en, ou consiste en, au moins un parmi, G*, σ_{c}, et EF, de préférence encore parmi EF, σ_{c}, et une combinaison quelconque de ceux-ci ; de préférence encore, le paramètre rhéologique déterminé/mesuré dans la zone non linéaire comprend au moins σ_{c}, ou consiste essentiellement en σ_{c}, ou est σ_{c}; et/ou
   ∘ le paramètre rhéologique déterminé/mesuré dans la zone linéaire est choisi parmi, et/ou comprend, ou consiste essentiellement en, ou consiste en, au moins un parmi, G', G", G*, γₚ, τₚ, et une combinaison quelconque de ceux-ci, de préférence le paramètre rhéologique mesuré dans la zone linéaire est choisi parmi, et/ou comprend, ou consiste essentiellement en, ou consiste en, au moins un parmi, G', G", G*, et une combinaison quelconque de ceux-ci; de préférence encore le paramètre rhéologique déterminé/mesuré dans la zone linéaire comprend au moins G*, ou consiste essentiellement en G*, ou est G* ;
- à un stade inférieur (i.e. plus léger/modéré) de la maladie que le ou les sujets de référence si au moins une des valeurs Aₗ et/ou Aₙₗ déterminées/mesurées est inférieure d'au moins 100% de la valeur limite du paramètre rhéologique, de préférence d'au moins 150%, de préférence d'au moins 200%, de préférence d'au moins 300%, de préférence d'au moins 400%, lorsque :
   ∘ le paramètre rhéologique déterminé/mesuré dans la zone non linéaire est choisi parmi, et/ou comprend, ou consiste essentiellement en, ou consiste en, au moins un parmi, G', G", G*, γ_{c}, τ_{c}, σ_{c}, EF, et une combinaison quelconque de ceux-ci , de préférence le paramètre rhéologique déterminé/mesuré dans la zone non linéaire est choisi parmi, et/ou comprend, ou consiste essentiellement en, ou consiste en, au moins un parmi, G', G", G*, σ_{c}, EF, et une combinaison quelconque de ceux-ci, de préférence encore le paramètre rhéologique déterminé/mesuré dans la zone non linéaire est choisi parmi, et/ou comprend, ou consiste essentiellement en, ou consiste en, au moins un parmi, G*, σ_{c}, et EF, de préférence encore parmi EF, σ_{c}, et une combinaison quelconque de ceux-ci ; de préférence encore, le paramètre rhéologique déterminé/mesuré dans la zone non linéaire comprend au moins σ_{c}, ou consiste essentiellement en σ_{c}, ou est σ_{c}; et/ou
   ∘ le paramètre rhéologique déterminé/mesuré dans la zone linéaire est choisi parmi, et/ou comprend, ou consiste essentiellement en, ou consiste en, au moins un parmi, G', G", G*, γₚ, τₚ, et une combinaison quelconque de ceux-ci, de préférence le paramètre rhéologique mesuré dans la zone linéaire est choisi parmi, et/ou comprend, ou consiste essentiellement en, ou consiste en, au moins un parmi, G', G", G*, et une combinaison quelconque de ceux-ci; de préférence encore le paramètre rhéologique déterminé/mesuré dans la zone linéaire comprend au moins G*, ou consiste essentiellement en G*, ou est G* ;
- à un stade supérieur (i.e. plus grave/avancé) de la maladie que le ou les sujets de référence si au moins une des valeurs Aₗ et/ou Aₙₗ déterminées/mesurées est inférieure d'au moins 10% de la valeur limite du paramètre rhéologique, de préférence d'au moins 15%, de préférence d'au moins 20%, de préférence d'au moins 30%, de préférence d'au moins 40%, de préférence d'au moins 50%, lorsque :
   ∘ le paramètre rhéologique déterminé/mesuré dans la zone non linéaire comprend au moins tan δ, ou consiste essentiellement en tan δ, ou est tan δ ; et/ou
   ∘ le paramètre rhéologique déterminé/mesuré dans la zone linéaire comprend au moins tan δ, ou consiste essentiellement en tan δ, ou est tan δ.

Les valeurs limites (ou gammes) peuvent être comme indiqué dans le Tableau 1 ci-dessous:
- selon un mode de réalisation particulier des méthodes selon l'invention, lorsque la valeur limite est la moyenne des valeurs d'un paramètre rhéologique déterminé/mesuré sur le mucus de plusieurs sujets sains de référence (au moins deux sujets sains de référence), et/ou
- selon un mode de réalisation particulier des méthodes selon l'invention, lorsque la valeur limite est la moyenne des valeurs d'un paramètre rhéologique déterminé/mesuré sur le mucus de plusieurs sujets de référence atteints de mucoviscidose (au moins deux sujets de référence), et/ou lorsque la valeur limite est la moyenne des valeurs d'un paramètre rhéologique déterminé/mesuré sur le mucus de plusieurs sujets de référence atteints de BPCO (au moins deux sujets de référence), et/ou
- selon un mode de réalisation particulier des méthodes selon l'invention, lorsque la valeur limite est la moyenne des valeurs d'un paramètre rhéologique déterminé/mesuré sur le mucus de plusieurs sujets de référence atteints d'asthme (au moins deux sujets de référence) :

**Tableau 1 : Valeur limite moyenne pour des sujets de référence atteints de mucoviscidose, des sujets de référence atteints de BPCO, pour des sujets de référence atteints d'asthme, et pour des sujets sains de référence, avec les écart-types, définissant ainsi une gamme.**

| **Valeur limite** | **Sujets de référence atteints de mucoviscidose** | **Sujets de référence atteints de BPCO** | **Sujets de référence atteints d'asthme** | **Sujets sains de référence** |
|---|---|---|---|---|
| **Paramètre rhéologique** | | | | |
| G' mesuré à 5 % de déformation (Pa) | 1,354 +/- 2,008 | 0,689 +/- 0,885 | 0,150 +/- 0,194 | 0,085 +/- 0,108 |
| G" mesuré à 5 % de déformation (Pa) | 0,508 +/- 0,716 | 0,234 +/- 0,175 | 0,067 +/- 0,082 | 0,0475 +/-0,057 |
| G'_{c} = G"_{c} mesuré au point d'écoulement c (Pa) | 0,230 +/- 0,161 | 0,195 +/- 0,147 | 0,066 +/- 0,055 | 0,044 +/- 0,028 |
| G* à 5 % de déformation (Pa) | 1,446 (allant de préférence de 1 à 2,5) | 0,728 (allant de préférence de 0,5 à 0,9) | 0,165 (allant de préférence de 0,15 à 0,3) | 0,0975 (allant de préférence de 0,09 à 0,1) |
| σ_{c} mesuré au point d'écoulement c (Pa) | 9,756 +/- 5,872 | 10,024 +/- 8,586 | 4,138 +/- 4,586 | 1,440 +/- 1,378 |
| τₚ mesuré à la limite supérieure du domaine linéaire (Pa) | 8,421 +/- 32,154 | 0,057 +/- 0,079 | 0,015 +/- 0,020 | 0,013 +/- 0,023 |
| tan δ dans le domaine linéaire | 0,275 +/- 0,072 | 0,303 +/- 0,103 | 0,378 +/- 0,120 | 0,361 +/- 0,110 |
| tan δ au point d'écoulement c | 1 | 1 | 1 | 1 par définition |
| EF (Pa²) | 3940,245 +/-14000 | 11,510 +/-21,453 | 1,321 +/- 1,624 | 0,292 +/- 0,488 |

Ainsi, dans un mode de réalisation, lorsque la valeur d'un paramètre rhéologique déterminé/mesuré sur le mucus du sujet est égale à la valeur limite ou tombe dans la gamme indiquée pour ce paramètre dans le tableau 1 pour des sujets de référence atteints de mucoviscidose (seconde colonne du tableau 1), cela indique que le sujet est atteint de mucoviscidose ; ou, de préférence, lorsque la valeur d'un paramètre rhéologique déterminé/mesuré sur le mucus du sujet est égale à la valeur limite ou tombe dans la gamme indiquée pour ce paramètre dans le tableau 1 pour des sujets de référence atteints de mucoviscidose (seconde colonne du tableau 1), la probabilité que le sujet soit atteint de mucoviscidose est élevée.

Dans un mode de réalisation, lorsque la valeur d'un paramètre rhéologique déterminé/mesuré sur le mucus du sujet est égale à la valeur limite indiquée ou tombe dans la gamme indiquée pour ce paramètre dans le tableau 1 pour des sujets de référence atteints de BPCO (troisième colonne du tableau 1), cela indique que le sujet est atteint de BPCO ; ou, de préférence, lorsque la valeur d'un paramètre rhéologique déterminé/mesuré sur le mucus du sujet est égale à la valeur limite ou tombe dans la gamme indiquée pour ce paramètre dans le tableau 1 pour des sujets de référence atteints de BPCO (troisième colonne du tableau 1), la probabilité que le sujet soit atteint de BPCO est élevée.

Dans un mode de réalisation, lorsque la valeur d'un paramètre rhéologique déterminé/mesuré sur le mucus du sujet est égale à la valeur limite ou tombe dans la gamme indiquée pour ce paramètre dans le tableau 1 pour des sujets de référence atteints d'asthme (quatrième colonne du tableau 1), cela indique que le sujet est atteint d'asthme ; ou, de préférence, lorsque la valeur d'un paramètre rhéologique déterminé/mesuré sur le mucus du sujet est égale à la valeur limite ou tombe dans la gamme indiquée pour ce paramètre dans le tableau 1 pour des sujets de référence atteints d'asthme (quatrième colonne du tableau 1), la probabilité que le sujet soit atteint d'asthme est élevée.

Selon un mode de réalisation préféré des méthodes selon l'invention, pour déterminer les valeurs limites, l'échantillon de mucus utilisé peut avoir été obtenu par expectoration spontanée dudit sujet. L'échantillon de mucus utilisé peut également avoir été obtenu par un prélèvement selon différentes techniques, incluant par exemple le lavage bronchiolo-alvéolaire (LBA), l'écouvillonnage pharyngé, l'aspiration pharyngée et l'expectoration induite, de préférence par expectoration induite.

Selon ce mode de réalisation préféré, la valeur limite est la moyenne des valeurs d'un paramètre rhéologique déterminé/mesuré sur le mucus de plusieurs sujets de référence (au moins deux sujets de référence), lesdits sujets étant soit des sujets sains, soit des sujets atteints de mucoviscidose, soit des sujets de référence atteints de BPCO ou de BPCO à un stade sévère, soit des sujets de référence atteints d'asthme ou d'asthme à un stade sévère, soit des sujets de référence atteints de dilatation des bronches (DDB). Selon ce mode de réalisation préféré, les valeurs limites (ou gammes) peuvent être comme indiqué dans le tableau 2, comme suit :

**Tableau 2 : Valeur limite moyenne pour des sujets de référence atteints de mucoviscidose, des sujets de référence atteints de BPCO ou de BPCO à un stade sévère, pour des sujets de référence atteints d'asthme ou d'asthme à un stade sévère, des sujets de référence atteints de dilatation des bronches (DDB), et pour des sujets sains de référence, avec les écart-types, définissant ainsi une gamme.**

| **Sujets de référence** | **Expectoration** | **G' (Pa)** | **G" (Pa)** | **G* (Pa)** | **tan 8 (-)** | **σ_{c} (Pa)** | **EF (Pa²)** |
|---|---|---|---|---|---|---|---|
| Sain | Induite | 0,14 | 0,047 | 0,15 | 0,35 | 0,012 | 0,27 |
| min-max | | 0.07-0.28 | 0.010-0.129 | 0.07-0.28 | 0.14-0.52 | 0.005-0.036 | 0.14-0.62 |
| Asthme | Induite | 0,25 | 0,098 | 0,27 | 0,41 | 0,019 | 0,75 |
| min-max | | 0.07-0.59 | 0.043-0.208 | 0.07-0.59 | 0.22-0.63 | 0.007-0.068 | 0.23-2.81 |
| BPCO | Induite | 0,43 | 0,14 | 0,45 | 0,34 | 0,097 | 3,61 |
| min-max | | 0.09-1.88 | 0.03-0.44 | 0.09-1.88 | 0.21-0.48 | 0.024-0.327 | 0.87-11.91 |
| BPCO | spontané | 1,15 | 0,33 | 1,2 | 0,31 | 0,31 | 11,77 |
| min-max | | 0.10-5.54 | 0.05-1.12 | 0.10-5.54 | 0.20-0.52 | 0.03-1.08 | 1.05-38.90 |
| Mucovisci dose | Induite | 0,84 | 0,28 | 0,89 | 0,35 | 0,21 | 6,34 |
| min-max | | 0.21-3.83 | 0.09-0.76 | 0.21-3.83 | 0.23-0.51 | 0.02-0.59 | 1.03-19.33 |
| Mucoviscidose | spontané | 1,24 | 0,41 | 1,31 | 0,34 | 0,32 | 9,68 |
| min-max | | 0.23-3.41 | 0.12-1.17 | 0.23-3.41 | 0.28-0.53 | 0.04-0.76 | 1.43-26.00 |
| Asthme sévère | spontané | 12,62 | 2,64 | 12,89 | 0,24 | 63,08 | 813 |
| Ecart-type | | 10,06 | 1,74 | 10,33 | 0,06 | 62,86 | 649 |
| BPCO sévère | spontané | 3,01 | 0,85 | 3,13 | 0,34 | 24,1 | 75,4 |
| Ecart-type | | 1,43 | 0,18 | 1,42 | 0,16 | 11,92 | 16,9 |
| DDB | spontané | 2,8 | 0,87 | 2,93 | 0,32 | 36,19 | 106 |
| Ecart-type | | 1,83 | 0,45 | 1,86 | 0,08 | 21,4 | 39,8 |

Ainsi, dans ce mode de réalisation, lorsque la valeur d'un paramètre rhéologique déterminé/mesuré sur le mucus du sujet est égale à la valeur limite ou tombe dans la gamme indiquée pour ce paramètre dans le tableau 2 pour des sujets de référence atteints de mucoviscidose, cela indique que le sujet est atteint de mucoviscidose ; ou, de préférence, lorsque la valeur d'un paramètre rhéologique déterminé/mesuré sur le mucus du sujet est égale à la valeur limite ou tombe dans la gamme indiquée pour ce paramètre dans le tableau 2 pour des sujets de référence atteints de mucoviscidose, la probabilité que le sujet soit atteint de mucoviscidose est élevée.

Dans ce mode de réalisation, lorsque la valeur d'un paramètre rhéologique déterminé/mesuré sur le mucus du sujet est égale à la valeur limite indiquée ou tombe dans la gamme indiquée pour ce paramètre dans le tableau 2 pour des sujets de référence atteints de BPCO, cela indique que le sujet est atteint de BPCO ; ou, de préférence, lorsque la valeur d'un paramètre rhéologique déterminé/mesuré sur le mucus du sujet est égale à la valeur limite ou tombe dans la gamme indiquée pour ce paramètre dans le tableau 2 pour des sujets de référence atteints de BPCO, la probabilité que le sujet soit atteint de BPCO est élevée.

Dans un mode de réalisation, lorsque la valeur d'un paramètre rhéologique déterminé/mesuré sur le mucus du sujet est égale à la valeur limite ou tombe dans la gamme indiquée pour ce paramètre dans le tableau 2 pour des sujets de référence atteints d'asthme, cela indique que le sujet est atteint d'asthme ; ou, de préférence, lorsque la valeur d'un paramètre rhéologique déterminé/mesuré sur le mucus du sujet est égale à la valeur limite ou tombe dans la gamme indiquée pour ce paramètre dans le tableau 2 pour des sujets de référence atteints d'asthme, la probabilité que le sujet soit atteint d'asthme est élevée.

Selon un mode de réalisation, les méthodes selon l'invention comprennent en outre la détermination ou la mesure de la valeur Bₗ d'au moins un paramètre rhéologique (tel que défini ci-dessus) sur un second échantillon de mucus (B) dudit sujet, dans une zone linéaire de la courbe de déformation de l'échantillon ; et/ou la détermination ou la mesure Bₙₗ d'au moins un paramètre rhéologique (tel que défini ci-dessus) sur le second échantillon (B), dans une zone non linéaire de la courbe de déformation de l'échantillon ; ledit second échantillon (B) ayant été obtenu/prélevé postérieurement à l'échantillon (A), de préférence ledit échantillon (B) ayant été obtenu au moins 24 heures après l'échantillon (A), de préférence encore au moins 48 heures après l'échantillon (A), de préférence encore au moins 72 heures après l'échantillon (A), de préférence encore au moins 7 jours après l'échantillon (A), de préférence encore au moins 10 jours après l'échantillon (A), de préférence encore au moins 15 jours après l'échantillon (A), de préférence encore au moins 1 mois après l'échantillon (A), de préférence encore au moins 2 mois après l'échantillon (A), de préférence encore au moins 3 mois après l'échantillon (A), de préférence encore au moins 4 mois après l'échantillon (A), de préférence encore au moins 5 mois après l'échantillon (A), , de préférence encore au moins 6 mois après l'échantillon (A), de préférence encore au moins 7 mois après l'échantillon (A), de préférence encore au moins 8 mois après l'échantillon (A), de préférence encore au moins 9 mois après l'échantillon (A), de préférence encore au moins 10 mois après l'échantillon (A), de préférence encore au moins 11 mois après l'échantillon (A), de préférence encore au moins 12 mois après l'échantillon (A). De préférence encore ledit échantillon (B) a été obtenu entre 7 jours et 6 mois après l'échantillon (A), de préférence encore ledit échantillon (B) ayant été obtenu entre 10 jours et 5 mois après l'échantillon (A), de préférence encore entre 15 jours et 4 mois, de préférence encore entre 21 jours et 3 mois, de préférence encore entre 30 et 60, de préférence encore entre 40 et 50 jours.

Selon un mode de réalisation, la méthode comprend en outre la détermination de l'évolution Eₗ de la valeur du paramètre rhéologique déterminé/mesuré dans la zone linéaire de la courbe de déformation et/ou l'évolution Eₙₗ de la valeur du paramètre rhéologique déterminé/mesuré dans la zone linéaire de la courbe de déformation. Avantageusement, l'évolution Eₗ de la valeur du paramètre rhéologique déterminé/mesuré dans la zone linéaire de la courbe de déformation est déterminée en comparant la valeur Bₗ à la valeur Aₗ et/ou l'évolution Eₙₗ de la valeur du paramètre rhéologique déterminé/mesuré dans la zone linéaire de la courbe de déformation est déterminée en comparant la valeur Bₙₗ à la valeur Aₙₗ.

Dans ce cas, le sujet est atteint d'une maladie des poumons et/ou d'une maladie respiratoire, ou le pronostic d'une maladie des poumons et/ou d'une maladie respiratoire est négatif, ou une maladie des poumons et/ou une maladie respiratoire est exacerbée, ou le traitement d'une maladie des poumons et/ou d'une maladie respiratoire est inefficace ou peu efficace, si la valeur Bₗ déterminée/mesurée est différente de la valeur Aₗ déterminée/mesurée pour au moins un paramètre rhéologique et/ou si la valeur Bₙₗ déterminée/mesurée est différente de la valeur Aₙₗ déterminée/mesurée pour au moins un paramètre rhéologique.

En particulier, le sujet est atteint d'une maladie des poumons et/ou d'une maladie respiratoire, ou le pronostic d'une maladie des poumons et/ou d'une maladie respiratoire est négatif, ou une maladie des poumons et/ou une maladie respiratoire est exacerbée, ou le traitement d'une maladie des poumons et/ou d'une maladie respiratoire est inefficace ou peu efficace, si au moins une des valeurs Aₗ déterminées/mesurées est supérieure à la valeur Bₗ déterminée/mesurée pour le même paramètre rhéologique, et/ou si au moins une des valeurs Aₙₗ déterminées/mesurées est supérieure à la valeur Bₙₗ déterminée/mesurée pour le même paramètre rhéologique, lorsque :
- le paramètre rhéologique déterminé/mesuré dans la zone non linéaire est choisi parmi, et/ou comprend, ou consiste essentiellement en, ou consiste en, au moins un parmi, G', G", G*, γ_{c}, τ_{c}, σ_{c}, EF, et une combinaison quelconque de ceux-ci , de préférence le paramètre rhéologique déterminé/mesuré dans la zone non linéaire est choisi parmi, et/ou comprend, ou consiste essentiellement en, ou consiste en, au moins un parmi, G', G", G*, σ_{c}, EF, et une combinaison quelconque de ceux-ci, de préférence encore le paramètre rhéologique déterminé/mesuré dans la zone non linéaire est choisi parmi, et/ou comprend, ou consiste essentiellement en, ou consiste en, au moins un parmi, G*, σ_{c}, et EF, de préférence encore parmi EF, σ_{c}, et une combinaison quelconque de ceux-ci ; de préférence encore, le paramètre rhéologique déterminé/mesuré dans la zone non linéaire comprend au moins σ_{c}, ou consiste essentiellement en σ_{c}, ou est σ_{c}; et/ou
- le paramètre rhéologique déterminé/mesuré dans la zone linéaire est choisi parmi, et/ou comprend, ou consiste essentiellement en, ou consiste en, au moins un parmi, G', G", G*, γₚ, τₚ, et une combinaison quelconque de ceux-ci, de préférence le paramètre rhéologique mesuré dans la zone linéaire est choisi parmi, et/ou comprend, ou consiste essentiellement en, ou consiste en, au moins un parmi, G', G", G*, et une combinaison quelconque de ceux-ci; de préférence encore le paramètre rhéologique déterminé/mesuré dans la zone linéaire comprend au moins G*, ou consiste essentiellement en G*, ou est G*.

A l'inverse, selon un mode de réalisation préféré, le sujet n'est pas atteint d'une maladie des poumons et/ou d'une maladie respiratoire, ou le pronostic d'une maladie des poumons et/ou d'une maladie respiratoire est positif, ou une maladie des poumons et/ou une maladie respiratoire n'est pas exacerbée, ou le traitement d'une maladie des poumons et/ou d'une maladie respiratoire est efficace, si au moins une des valeurs Aₗ déterminées/mesurées est inférieure à la valeur Bₗ déterminée/mesurée pour le même paramètre rhéologique, et/ou si au moins une des valeurs Aₙₗ déterminées/mesurées est inférieure à la valeur Bₙₗ déterminée/mesurée pour le même paramètre rhéologique, lorsque :
- le paramètre rhéologique déterminé/mesuré dans la zone non linéaire est choisi parmi, et/ou comprend, ou consiste essentiellement en, ou consiste en, au moins un parmi, G', G", G*, γ_{c}, τ_{c}, σ_{c}, EF, et une combinaison quelconque de ceux-ci , de préférence le paramètre rhéologique déterminé/mesuré dans la zone non linéaire est choisi parmi, et/ou comprend, ou consiste essentiellement en, ou consiste en, au moins un parmi, G', G", G*, σ_{c}, EF, et une combinaison quelconque de ceux-ci, de préférence encore le paramètre rhéologique déterminé/mesuré dans la zone non linéaire est choisi parmi, et/ou comprend, ou consiste essentiellement en, ou consiste en, au moins un parmi, G*, σ_{c}, et EF, de préférence encore parmi EF, σ_{c}, et une combinaison quelconque de ceux-ci ; de préférence encore, le paramètre rhéologique déterminé/mesuré dans la zone non linéaire comprend au moins σ_{c}, ou consiste essentiellement en σ_{c}, ou est σ_{c}; et/ou
- le paramètre rhéologique déterminé/mesuré dans la zone linéaire est choisi parmi, et/ou comprend, ou consiste essentiellement en, ou consiste en, au moins un parmi, G', G", G*, γₚ, τₚ, et une combinaison quelconque de ceux-ci, de préférence le paramètre rhéologique mesuré dans la zone linéaire est choisi parmi, et/ou comprend, ou consiste essentiellement en, ou consiste en, au moins un parmi, G', G", G*, et une combinaison quelconque de ceux-ci; de préférence encore le paramètre rhéologique déterminé/mesuré dans la zone linéaire comprend au moins G*, ou consiste essentiellement en G*, ou est G*.

Selon un mode de réalisation préféré, le sujet est atteint d'une maladie des poumons et/ou d'une maladie respiratoire, ou le pronostic d'une maladie des poumons et/ou d'une maladie respiratoire est négatif, ou une maladie des poumons et/ou une maladie respiratoire est exacerbée, ou le traitement d'une maladie des poumons et/ou d'une maladie respiratoire est inefficace ou peu efficace, si au moins une des valeurs Aₗ déterminées/mesurées est inférieure à la valeur Bₗ déterminée/mesurée pour le même paramètre rhéologique, et/ou si au moins une des valeurs Aₙₗ déterminées/mesurées est inférieure à la valeur Bₙₗ déterminée/mesurée pour le même paramètre rhéologique, lorsque :
- le paramètre rhéologique déterminé/mesuré dans la zone non linéaire comprend au moins tan δ, ou consiste essentiellement en tan δ, ou est tan δ; et/ou
- le paramètre rhéologique déterminé/mesuré dans la zone linéaire comprend au moins tan δ, ou consiste essentiellement en tan δ, ou est tan δ.

A l'inverse, selon un mode de réalisation préféré, le sujet n'est pas atteint d'une maladie des poumons et/ou d'une maladie respiratoire, ou le pronostic d'une maladie des poumons et/ou d'une maladie respiratoire est positif, ou une maladie des poumons et/ou une maladie respiratoire n'est pas exacerbée, ou le traitement d'une maladie des poumons et/ou d'une maladie respiratoire est efficace, si au moins une des valeurs Aₗ déterminées/mesurées est supérieure à la valeur Bₗ déterminée/mesurée pour le même paramètre rhéologique, et/ou si au moins une des valeurs Aₙₗ déterminées/mesurées est supérieure à la valeur Bₙₗ déterminée/mesurée pour le même paramètre rhéologique, lorsque :
- le paramètre rhéologique déterminé/mesuré dans la zone non linéaire comprend au moins tan δ, ou consiste essentiellement en tan δ, ou est tan δ; et/ou
- le paramètre rhéologique déterminé/mesuré dans la zone linéaire comprend au moins tan δ, ou consiste essentiellement en tan δ, ou est tan δ.

Selon un mode de réalisation particulier, le sujet est atteint d'une maladie des poumons et/ou d'une maladie respiratoire, ou le pronostic d'une maladie des poumons et/ou d'une maladie respiratoire est négatif, ou une maladie des poumons et/ou une maladie respiratoire est exacerbée, ou le traitement d'une maladie des poumons et/ou d'une maladie respiratoire est inefficace ou peu efficace, si l'évolution Eₗ de la valeur du paramètre rhéologique et/ou si l'évolution Eₙₗ de la valeur du paramètre rhéologique est différente d'une évolution seuil (ou évolution de référence ou évolution limite), de préférence dans laquelle ladite évolution seuil est choisie parmi : l'évolution de la valeur d'un paramètre rhéologique déterminé/mesuré sur le mucus d'un sujet sain de référence ; ou à la moyenne des évolutions de la valeur d'un paramètre rhéologique déterminées/mesurées sur des échantillons de mucus différents d'un même sujet sain de référence ou à la moyenne des évolutions de la valeur d'un paramètre rhéologique déterminé/mesuré sur le mucus de plusieurs sujets sains de référence (au moins deux sujets sains de référence) ; ou à l'évolution de la valeur d'un paramètre rhéologique déterminées/mesuré sur le mucus d'un sujet de référence à un stade connu de la maladie des poumons et/ou de la maladie respiratoire ; ou à la moyenne des évolutions de la valeur d'un paramètre rhéologique déterminées/mesurées sur des échantillons de mucus différents d'un même sujet de référence à un stade connu de la maladie des poumons et/ou de la maladie respiratoire ou à la moyenne des évolutions de la valeur d'un paramètre rhéologique déterminé/mesuré sur le mucus de plusieurs sujets de référence à un stade connu de la maladie des poumons et/ou de la maladie respiratoire (au moins deux sujets de référence à un stade connu de la maladie des poumons et/ou de la maladie respiratoire).

Selon un mode de réalisation des méthodes selon l'invention, le diagnostic, le pronostic la stratification et/ou le suivi de la maladie des poumons et/ou de la maladie respiratoire comprennent en outre l'évaluation de l'efficacité d'un traitement de la maladie des poumons et/ou de la maladie respiratoire administré audit sujet. L'efficacité d'un traitement de la maladie des poumons et/ou de la maladie respiratoire peut être évaluée en utilisant les méthodes connues de l'homme du métier. Selon un mode de réalisation avantageux, l'efficacité d'un traitement de la maladie des poumons et/ou de la maladie respiratoire est évaluée par la méthode *in vitro* d'évaluation de l'efficacité d'un traitement d'une maladie des poumons et/ou d'une maladie respiratoire selon la présente invention (telle que définie ci-dessus). Selon ce mode de réalisation, l'évaluation de l'efficacité du traitement est de préférence basée sur la valeur Aₙₗ, la valeur Aₗ, la valeur Bₙₗ, et/ou la valeur Bₗ, et/ou encore sur l'évolution Eₙₗ et ou l'évolution Eₗ. L'évaluation de l'efficacité d'un traitement de la maladie des poumons et/ou de la maladie respiratoire administré à un sujet est notamment effectuée comme décrit ci-dessus.

Avantageusement, les méthodes selon l'invention comprennent en outre une étape d'administration d'un traitement au sujet. L'homme du métier saura définir le traitement adapté, notamment le ou les médicament(s) adapté(s) et/ou la ou les thérapie(s) adaptée(s) à administrer au sujet et/ou à expérimenter, ainsi que le protocole à mettre en oeuvre et les modalités de l'administration, selon le sujet considéré, ainsi que le diagnostic, la stratification, le pronostic et/ou le suivi résultant de la mise en oeuvre d'une méthode selon l'invention (notamment selon la maladie respiratoire et/ou pulmonaire diagnostiquée et/ou stratifiée et/ou pronostiquée et/ou suivie). Avantageusement, le traitement est tel que défini ci-dessus.

De manière avantageuse, la maladie des poumons et/ou la maladie respiratoire est choisie parmi la BPCO, la mucoviscidose et l'asthme.

Les données obtenues par les Inventeurs montrent que le traitement préalable de patients à la rhDNase (désoxyribonucléase recombinante humaine) a un effet sur les paramètres rhéologiques du mucus du patient, révélant ainsi pour la première fois que la détermination ou la mesure de paramètres rhéologiques linéaires et non linéaires permet d'évaluer l'effet mucolytique d'un traitement. Ainsi, dans un mode de réalisation des méthodes selon la présente invention, le sujet est préalablement traité à la rhDNase, avant le prélèvement de l'échantillon de mucus. Avantageusement, la rhDNAse est administrée par nébulisation. Les modalités d'administration de la rhDNAse sont connues de l'homme du métier (notamment les doses, la durée de l'administration et le type de rhDNAse), en particulier selon le sujet considéré et la maladie respiratoire et/ou pulmonaire dont le sujet est atteint ou est susceptible d'être atteint.

Les données obtenues par les inventeurs sont d'autant plus pertinentes qu'elles ont été obtenues à l'aide d'un dispositif mis au point par les Inventeurs, permettant de simuler l'écoulement du mucus en situation physiologique. Les Inventeurs ont notamment montré que, grâce à la rugosité des surfaces planes en contact avec l'échantillon, ce dispositif permet d'obtenir de manière reproductible des mesures des propriétés viscoélastiques et rhéologiques du mucus placé en conditions physiologiques. Ainsi, selon un mode de réalisation des méthodes selon l'invention, la valeur (Aₙₗ), la valeur (Aₗ), la valeur (Bₙₗ), et/ou la valeur (Bₗ), sont mesurées à l'aide d'un appareil de mesure rhéologique dans lequel chaque échantillon est de préférence placé entre deux plaques mobiles en rotation l'une par rapport à l'autre, les surfaces en vis-à-vis desdites plaques étant de préférence rugueuses.

### Utilisations des paramètres rhéologiques en tant que biomarqueurs de maladies respiratoires : ne figure pas dans le texte des revendicaitons

Les Inventeurs ont ainsi montré que les paramètres rhéologiques mesurés sur un échantillon de mucus d'un sujet, comme décrit ci-dessus, peuvent être utilisés comme biomarqueurs d'une maladie respiratoire et/ou pulmonaire.

Il est donc également l'utilisation, qui ne figure pas dans le texte des revendications, ladite utilisation étant de préférence *in vitro,* d'au moins un paramètre rhéologique d'un échantillon de mucus A d'un sujet, déterminé ou mesuré dans une zone non linéaire de la courbe de déformation de l'échantillon ; et/ou d'au moins un paramètre rhéologique dudit échantillon de mucus A, déterminé ou mesuré dans une zone linéaire de la courbe de déformation de l'échantillon ; pour diagnostiquer, stratifier, pronostiquer et/ou suivre une maladie des poumons et/ou une maladie respiratoire ; dans laquelle le paramètre rhéologique est choisi parmi le module d'élasticité (G'), le module de viscosité (G"), le facteur d'amortissement (Tan δ), le module complexe (G*), la déformation plastique (γₚ), la déformation critique (γ_{c}), la contrainte plastique (τₚ), le seuil de contrainte à l'écoulement (τ_{c}) et la force élastique (EF) ;
le paramètre rhéologique déterminé ou mesuré dans la zone non linéaire étant de préférence choisi parmi G', G", G*, tan δ, γ_{c}, τ_{c}, EF, σ_{c}, et une combinaison quelconque de ceux-ci ; de préférence parmi G*, tan δ, τ_{c}, EF, σ_{c}, et une combinaison quelconque de ceux-ci ; de préférence encore parmi τ_{c}, EF, σ_{c}, et une combinaison quelconque de ceux-ci ; de préférence encore parmi τ_{c}, σ_{c}, et une combinaison quelconque de ceux-ci ; de préférence encore, le paramètre rhéologique déterminé ou mesuré dans la zone non linéaire comprend au moins σ_{c}; le paramètre rhéologique déterminé ou mesuré dans la zone linéaire étant de préférence choisi parmi G', G", G*, tan δ, γₚ, τₚ et une combinaison quelconque de ceux-ci ; de préférence parmi G*, tan δ, γₚ et une combinaison quelconque de ceux-ci ; de préférence encore parmi G*, tan δ et une combinaison quelconque de ceux-ci ; de préférence encore, le paramètre rhéologique déterminé ou mesuré dans la zone linéaire comprend au moins G*.

Il est également ici décrit l'utilisation, qui ne figure pas dans le texte des revendications, ladite utilisation étant de préférence *in vitro,* de la valeur Aₙₗ d'au moins un paramètre rhéologique déterminée ou mesurée sur un échantillon de mucus A d'un sujet, dans une zone non linéaire de la courbe de déformation de l'échantillon ; et/ou de la valeur Aₗ d'au moins un paramètre rhéologique déterminée ou mesurée sur ledit échantillon de mucus A, dans une zone linéaire de la courbe de déformation de l'échantillon ; pour diagnostiquer, stratifier, pronostiquer et/ou suivre une maladie des poumons et/ou une maladie respiratoire ; dans laquelle le paramètre rhéologique est choisi parmi le module d'élasticité (G'), le module de viscosité (G"), le facteur d'amortissement (Tan δ), le module complexe (G*), la déformation plastique (γₚ), la déformation critique (γ_{c}), la contrainte plastique (τₚ), le seuil de contrainte à l'écoulement (τ_{c}), la force élastique (EF), la contrainte critique (σ_{c}), et une combinaison quelconque de ceux-ci ;
le paramètre rhéologique déterminé ou mesuré dans la zone non linéaire étant de préférence choisi parmi G', G", G*, tan δ, γ_{c}, τ_{c}, EF, σ_{c}, et une combinaison quelconque de ceux-ci ; de préférence parmi G*, tan δ, τ_{c}, EF, σ_{c}, et une combinaison quelconque de ceux-ci ; de préférence encore parmi τ_{c}, EF, σ_{c}, et une combinaison quelconque de ceux-ci ; de préférence encore parmi τ_{c}, σ_{c}, et une combinaison quelconque de ceux-ci ; de préférence encore, le paramètre rhéologique déterminé ou mesuré dans la zone non linéaire comprend au moins σ_{c}; le paramètre rhéologique déterminé ou mesuré dans la zone linéaire étant de préférence choisi parmi G', G", G*, tan δ, γₚ, τₚ et une combinaison quelconque de ceux-ci ; de préférence parmi G*, tan δ, γₚ et une combinaison quelconque de ceux-ci ; de préférence encore parmi G*, tan δ et une combinaison quelconque de ceux-ci ; de préférence encore, le paramètre rhéologique déterminé ou mesuré dans la zone linéaire comprend au moins G*.

Les Inventeurs ont ainsi montré que les paramètres rhéologiques mesurés sur un échantillon de mucus d'un sujet, comme décrit ci-dessus, peuvent être utilisés comme biomarqueurs pour évaluer l'efficacité d'un traitement d'une maladie respiratoire et/ou pulmonaire.

Il est donc également ici décrit l'utilisation, qui ne figure pas dans le texte des revendications, ladite utilisation étant de préférence *in vitro,* d'au moins un paramètre rhéologique d'un échantillon de mucus A d'un sujet souffrant d'une maladie des poumons et/ou d'une maladie respiratoire et ayant reçu au moins une administration d'un traitement, déterminé ou mesuré dans une zone non linéaire de la courbe de déformation de l'échantillon ; et/ou d'au moins un paramètre rhéologique dudit échantillon de mucus A, déterminé ou mesuré dans une zone linéaire de la courbe de déformation de l'échantillon ; pour évaluer l'efficacité dudit traitement de ladite maladie des poumons et/ou de ladite maladie respiratoire ; dans laquelle le paramètre rhéologique est choisi parmi le module d'élasticité (G'), le module de viscosité (G"), le facteur d'amortissement (Tan δ), le module complexe (G*), la déformation plastique (γₚ), la déformation critique (γ_{c}), la contrainte plastique (τₚ), le seuil de contrainte à l'écoulement (τ_{c}), la force élastique (EF), la contrainte critique (σ_{c}), et une combinaison quelconque de ceux-ci ;
le paramètre rhéologique déterminé ou mesuré dans la zone non linéaire étant de préférence choisi parmi G', G", G*, tan δ, γ_{c}, τ_{c}, EF, σ_{c}, et une combinaison quelconque de ceux-ci ; de préférence parmi G*, tan δ, τ_{c}, EF, σ_{c}, et une combinaison quelconque de ceux-ci ; de préférence encore parmi τ_{c}, EF, σ_{c}, et une combinaison quelconque de ceux-ci ; de préférence encore parmi τ_{c}, σ_{c}, et une combinaison quelconque de ceux-ci ; de préférence encore, le paramètre rhéologique déterminé ou mesuré dans la zone non linéaire comprend au moins σ_{c}; le paramètre rhéologique déterminé ou mesuré dans la zone linéaire étant de préférence choisi parmi G', G", G*, tan δ, γₚ, τₚ et une combinaison quelconque de ceux-ci ; de préférence parmi G*, tan δ, γₚ et une combinaison quelconque de ceux-ci ; de préférence encore parmi G*, tan δ et une combinaison quelconque de ceux-ci ; de préférence encore, le paramètre rhéologique déterminé ou mesuré dans la zone linéaire comprend au moins G*.

Il est également ici décrit l'utilisation, qui ne figure pas dans le texte des revendications, ladite utilisation étant de préférence *in vitro,* de la valeur Aₙₗ d'au moins un paramètre rhéologique déterminée ou mesurée sur un échantillon de mucus A d'un sujet souffrant d'une maladie des poumons et/ou d'une maladie respiratoire et ayant reçu au moins une administration d'un traitement, dans une zone non linéaire de la courbe de déformation de l'échantillon ; et/ou de la valeur Aₗ d'au moins un paramètre rhéologique déterminée ou mesurée sur ledit échantillon de mucus A, dans une zone linéaire de la courbe de déformation de l'échantillon ; pour évaluer l'efficacité dudit traitement de ladite maladie des poumons et/ou de ladite maladie respiratoire ; dans laquelle le paramètre rhéologique est choisi parmi le module d'élasticité (G'), le module de viscosité (G"), le facteur d'amortissement (Tan δ), le module complexe (G*), la déformation plastique (γₚ), la déformation critique (γ_{c}), la contrainte plastique (τₚ), le seuil de contrainte à l'écoulement (τ_{c}), la force élastique (EF), la contrainte critique (σ_{c}), et une combinaison quelconque de ceux-ci ;
le paramètre rhéologique déterminé ou mesuré dans la zone non linéaire étant de préférence choisi parmi G', G", G*, tan δ, γ_{c}, τ_{c}, EF, σ_{c}, et une combinaison quelconque de ceux-ci ; de préférence parmi G*, tan δ, τ_{c}, EF, σ_{c}, et une combinaison quelconque de ceux-ci ; de préférence encore parmi τ_{c}, EF, σ_{c}, et une combinaison quelconque de ceux-ci ; de préférence encore parmi τ_{c}, σ_{c}, et une combinaison quelconque de ceux-ci ; de préférence encore, le paramètre rhéologique déterminé ou mesuré dans la zone non linéaire comprend au moins σ_{c}; le paramètre rhéologique déterminé ou mesuré dans la zone linéaire étant de préférence choisi parmi G', G", G*, tan δ, γₚ, τₚ et une combinaison quelconque de ceux-ci ; de préférence parmi G*, tan δ, γₚ et une combinaison quelconque de ceux-ci ; de préférence encore parmi G*, tan δ et une combinaison quelconque de ceux-ci ; de préférence encore, le paramètre rhéologique déterminé ou mesuré dans la zone linéaire comprend au moins G*.

Il est également ici décrit l'utilisation, qui ne figure pas dans le texte des revendications, ladite utilisation étant de préférence *in vitro,* de la valeur Aₙₗ d'au moins un paramètre rhéologique déterminée ou mesurée sur un échantillon de mucus A d'un sujet, dans une zone non linéaire de la courbe de déformation de l'échantillon ; et/ou de la valeur Aₗ d'au moins un paramètre rhéologique déterminée ou mesurée sur ledit échantillon de mucus A, dans une zone linéaire de la courbe de déformation de l'échantillon ; comme biomarqueur pour diagnostiquer, stratifier, pronostiquer et/ou suivre une maladie des poumons et/ou une maladie respiratoire ; et/ou comme biomarqueur pour évaluer l'efficacité d'un traitement d'une maladie des poumons et/ou une maladie respiratoire (dans ce cas ledit sujet souffre de la maladie des poumons et/ou de la maladie respiratoire et a reçu au moins une administration dudit traitement) ;
dans laquelle le paramètre rhéologique est choisi parmi le module d'élasticité (G'), le module de viscosité (G"), le facteur d'amortissement (Tan δ), le module complexe (G*), la déformation plastique (γₚ), la déformation critique (γ_{c}), la contrainte plastique (τₚ), le seuil de contrainte à l'écoulement (τ_{c}), la force élastique (EF), la contrainte critique (σ_{c}), et une combinaison quelconque de ceux-ci ;
le paramètre rhéologique déterminé ou mesuré dans la zone non linéaire étant de préférence choisi parmi G', G", G*, tan δ, γ_{c}, τ_{c}, EF, σ_{c}, et une combinaison quelconque de ceux-ci ; de préférence parmi G*, tan δ, τ_{c}, EF, σ_{c}, et une combinaison quelconque de ceux-ci ; de préférence encore parmi τ_{c}, EF, σ_{c}, et une combinaison quelconque de ceux-ci ; de préférence encore parmi τ_{c}, σ_{c}, et une combinaison quelconque de ceux-ci ; de préférence encore, le paramètre rhéologique déterminé ou mesuré dans la zone non linéaire comprend au moins σ_{c}; le paramètre rhéologique déterminé ou mesuré dans la zone linéaire étant de préférence choisi parmi G', G", G*, tan δ, γₚ, τₚ et une combinaison quelconque de ceux-ci ; de préférence parmi G*, tan δ, γₚ et une combinaison quelconque de ceux-ci ; de préférence encore parmi G*, tan δ et une combinaison quelconque de ceux-ci ; de préférence encore, le paramètre rhéologique déterminé ou mesuré dans la zone linéaire comprend au moins G*.

### Test compagnon : ne figure pas dans le texte des revendications

Il est en outre ici décrit un test compagnon, qui ne figure pas dans le texte des revendications, ledit test compagnon comprenant :
(i) la détermination ou la mesure de la valeur Aₙₗ d'au moins un paramètre rhéologique sur un échantillon de mucus A d'un sujet, dans une zone non linéaire de la courbe de déformation de l'échantillon ; et/ou
(ii) la détermination ou la mesure de la valeur Aₗ d'au moins un paramètre rhéologique sur ledit échantillon de mucus A, dans une zone linéaire de la courbe de déformation de l'échantillon ;
dans lequel le paramètre rhéologique est choisi parmi :
- le module d'élasticité (G'),
- le module de viscosité (G"),
- le facteur d'amortissement (tan δ),
- le module complexe (G*),
- la déformation plastique (γₚ),
- la déformation critique (γ_{c}),
- la contrainte plastique (τₚ),
- le seuil de contrainte à l'écoulement (τ_{c}),
- la force élastique (EF),
- la contrainte critique (σ_{c}), et
- une combinaison quelconque de ceux-ci ;
le paramètre rhéologique déterminé ou mesuré dans la zone non linéaire étant de préférence choisi parmi G', G", G*, tan δ, γ_{c}, τ_{c}, EF, σ_{c}, et une combinaison quelconque de ceux-ci ; de préférence parmi G*, tan δ, τ_{c}, EF, σ_{c}, et une combinaison quelconque de ceux-ci ; de préférence encore parmi τ_{c}, EF, σ_{c}, et une combinaison quelconque de ceux-ci ; de préférence encore parmi τ_{c}, σ_{c}, et une combinaison quelconque de ceux-ci ; de préférence encore, le paramètre rhéologique déterminé ou mesuré dans la zone non linéaire comprend au moins σ_{c}; le paramètre rhéologique déterminé ou mesuré dans la zone linéaire étant de préférence choisi parmi G', G", G*, tan δ, γₚ, τₚ et une combinaison quelconque de ceux-ci ; de préférence parmi G*, tan δ, γₚ et une combinaison quelconque de ceux-ci ; de préférence encore parmi G*, tan δ et une combinaison quelconque de ceux-ci ; de préférence encore, le paramètre rhéologique déterminé ou mesuré dans la zone linéaire comprend au moins G*.

Avantageusement, les paramètres rhéologiques des utilisations ici décrites et/ou du test compagnon ici décrit, ainsi que leur détermination/mesure, le sujet et l'échantillon de mucus, sont tels que définis ci-dessus en lien avec la méthode *in vitro* de diagnostic, de stratification, de pronostic et/ou de suivi d'une maladie des poumons et/ou une maladie respiratoire et/ou avec la méthode *in vitro* d'évaluation de l'efficacité d'un traitement d'une maladie des poumons et/ou d'une maladie respiratoire.

Par exemple, la valeur Aₗ et/ou la valeur Aₙₗ déterminée/mesurée est comparée à une valeur limite. Avantageusement, cette valeur limite est telle que définie ci-dessus en lien avec la méthode *in vitro* de diagnostic, de stratification, de pronostic et/ou de suivi d'une maladie des poumons et/ou une maladie respiratoire et/ou avec la méthode *in vitro* d'évaluation de l'efficacité d'un traitement d'une maladie des poumons et/ou d'une maladie respiratoire. En outre, l'utilisation de cette valeur limite, sa comparaison avec la valeur Aₗ et/ou la valeur Aₙₗ déterminée/mesurée, et/ou les conclusion qui en sont tirées quant à l'état du patient, sont de préférence tels que définis ci-dessus en lien avec la méthode in vitro de diagnostic, de stratification, de pronostic et/ou de suivi d'une maladie des poumons et/ou une maladie respiratoire et/ou avec la méthode in vitro d'évaluation de l'efficacité d'un traitement d'une maladie des poumons et/ou d'une maladie respiratoire.

### EXEMPLES

### 1. ETUDE CLINIQUE N°1 : Sujets atteints de mucoviscidose, de BPCO ou d'asthme

### 1.1. Matériels et Méthodes

Le protocole clinique NCT02682290 a été mené par le CHU Grenoble-Alpes (CHUGA). Quatre groupes de patients ont été retenus : 11 sujets sains (HV, pour « Healthy Volunteers » en anglais), 11 atteints de mucoviscidose (CF, pour « Cystic Fibrosis » en anglais), 11 de broncho pneumopathie chronique obstructive (BPCO, ou COPD, pour « Chronic obstructive pulmonary disease » en anglais) et 12 asthmatiques (AS ou Ast, pour « Asthma » en anglais).

Critères d'inclusion : patients Muco (atteints de mucoviscidose), BPCO (toutes classes), asthmatiques avec affection bronchique confirmée par le CHUGA. Pour les volontaires sains : homme ou femme de plus de 18 ans, Indice de Masse Corporelle (IMC) > 18 et < 29, non-fumeur, absence de toute pathologie aiguë dans le mois précédent, personne affiliée à la sécurité sociale ou bénéficiaire d'un tel régime.

Critères d'exclusion : FEV1 ≤ 40%, PaO2 < 60 mmHg au repos, exacerbation aiguë durant le mois précédent, contrindications pour la spirométrie.

Deux visites V1 et V2 ont été menées à 48 h d'intervalle. Les patients CF et COPD expectorent spontanément, pour les HV et Ast l'expectoration est induite par solution saline 4.5 % pendant 10 min (protocole standard). La salive est séparée et l'échantillon est homogénéisé suivant le protocole décrit dans WO/2019/020932 (Method for Processing a Sputum Sample and Method for the Rheometric Measurement of a Preprocessed Sample of This Kind). Le premier échantillon expectoré est séparé en deux, l'un pour mesure immédiate, l'autre pour une répétition à 10 min.

Les tests rhéologiques sont réalisés à 37°C avec l'appareil Rheomuco (Rheonova, France). Rheomuco est un rhéomètre rotatif plan-plan à déformation imposée. Les plateaux au contact du mucus sont rendus rugueux (arrangement régulier de pyramides à base carrée, de base 0,5 mm et de hauteur 0,25 mm) pour garantir l'adhésion jusqu'au point d'écoulement.

Une séquence d'oscillations à 1 Hz est appliquée avec une amplitude croissante par paliers de 0.1 à 3000 % afin d'extraire les propriétés rhéologiques du mucus dans les domaines linéaire (petites déformations, typiquement < 20 %) et non linéaire (grandes déformations).

Le protocole suivi est identique pour la V2, sauf pour les CF : l'expectoration spontanée initiale est suivie d'une nébulisation de rhDNase pendant 20 min, puis d'une nouvelle expectoration spontanée 1h plus tard.

### 1.2. Résultats

Les valeurs p sont données en comparaison aux sujets sains.

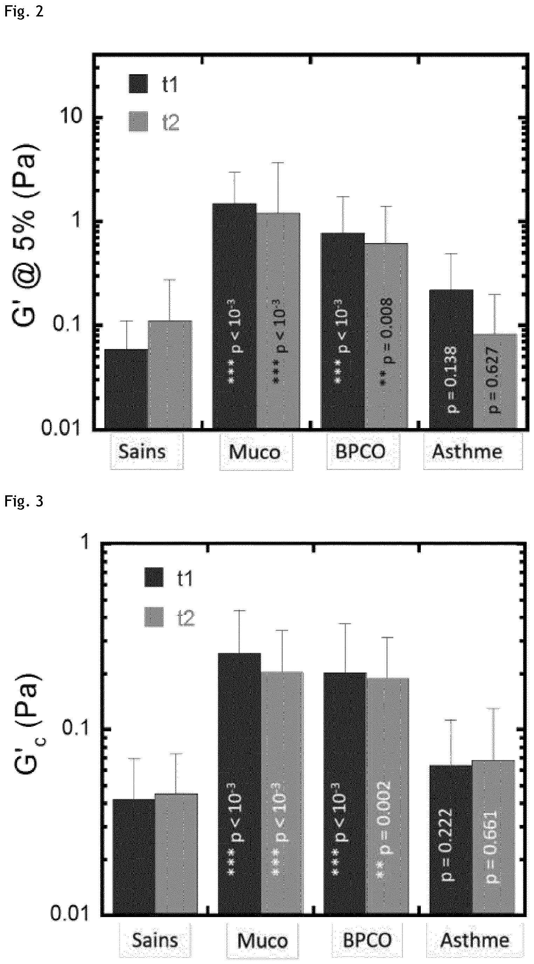

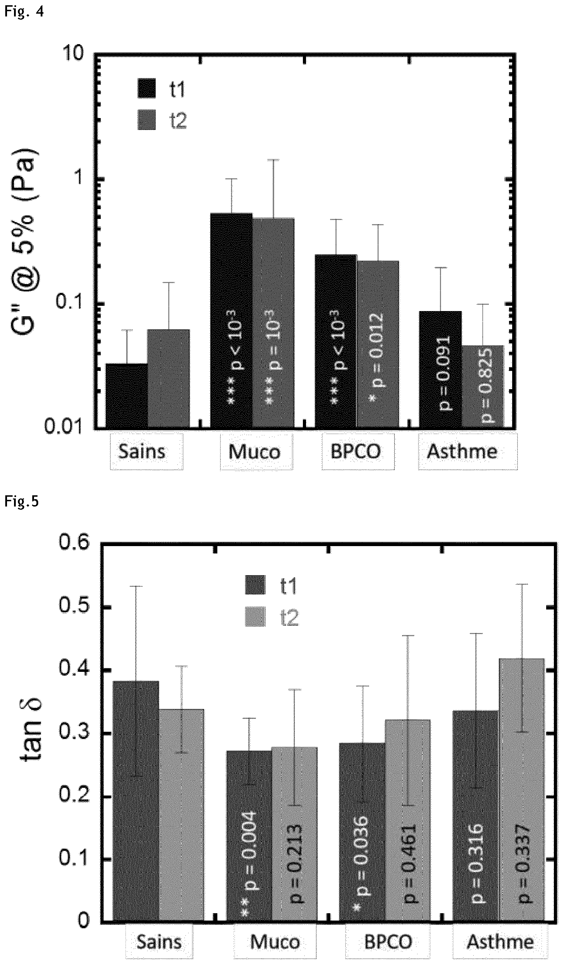

Les données montrent que les modules G', G" et G* mesurés dans la zone linéaire de la courbe de déformation (à 5% de déformation) sont des marqueurs sélectifs : les 3 pathologies sont nettement discriminées. La répétabilité à 10 min est très bonne (Figures 2 et 4 et tableaux 3 et 4).

Le marqueur tan δ (facteur d'amortissement) montre une légère baisse pour les patients souffrant de mucoviscidose ou de BPCO comparativement aux sujets sains, statistiquement significative mais pas au regard de la variabilité de l'échantillon. La répétabilité à 10 min est bonne (Figure 5 et tableaux 3 et 4).

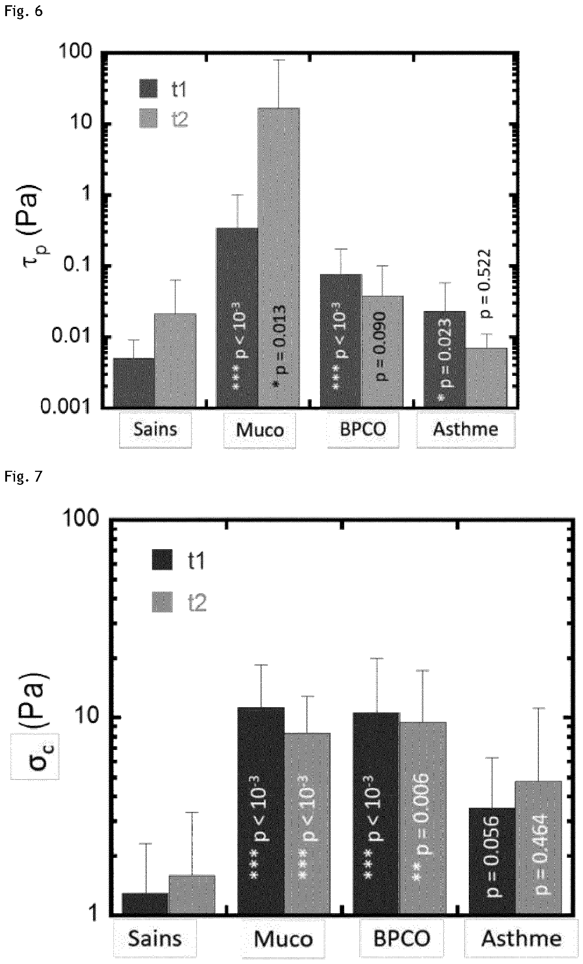

Le module G'_{c} et la contrainte seuil τ_{c} (seuil de contrainte à l'écoulement) mesurés au point d'écoulement c de la courbe de déformation distinguent assez nettement les états sain et pathologiques, malgré un léger recouvrement des patients atteints de mucoviscidose ou de BPCO (Figures 3 et 7 et tableaux 3 et 4). La contrainte plastique τₚ mesurée à la limite supérieure de la zone linéaire montre également une bonne discrimination des pathologies. Toutefois, la répétabilité est moins bonne (Figure 6 et tableaux 3 et 4).

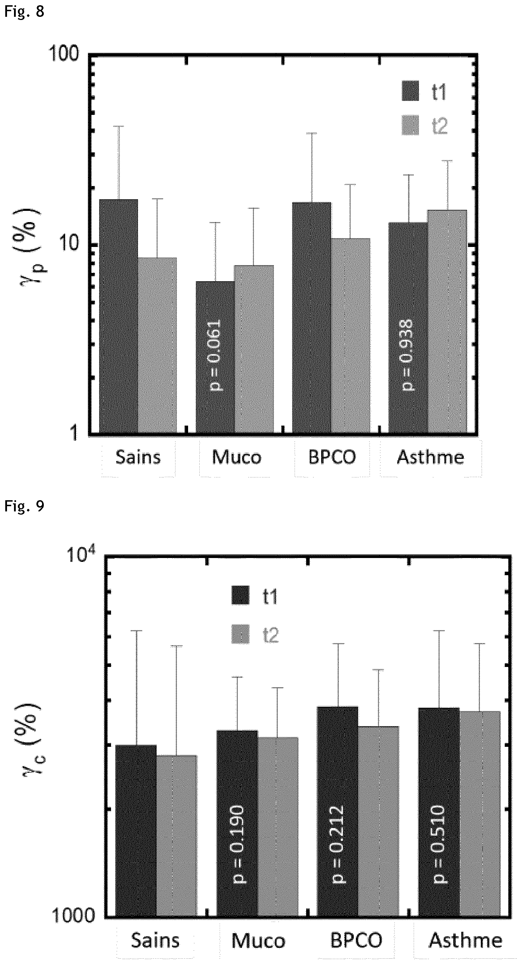

La déformation γ correspondant aux transitions entre régimes est globalement indépendante de l'état pathologique, aussi bien à la limite supérieure de la zone linéaire (γₚ) qu'au point d'écoulement c (γ_{c}). La répétabilité à 10 min est très bonne (Figures 8 et 9 et tableaux 3 et 4).

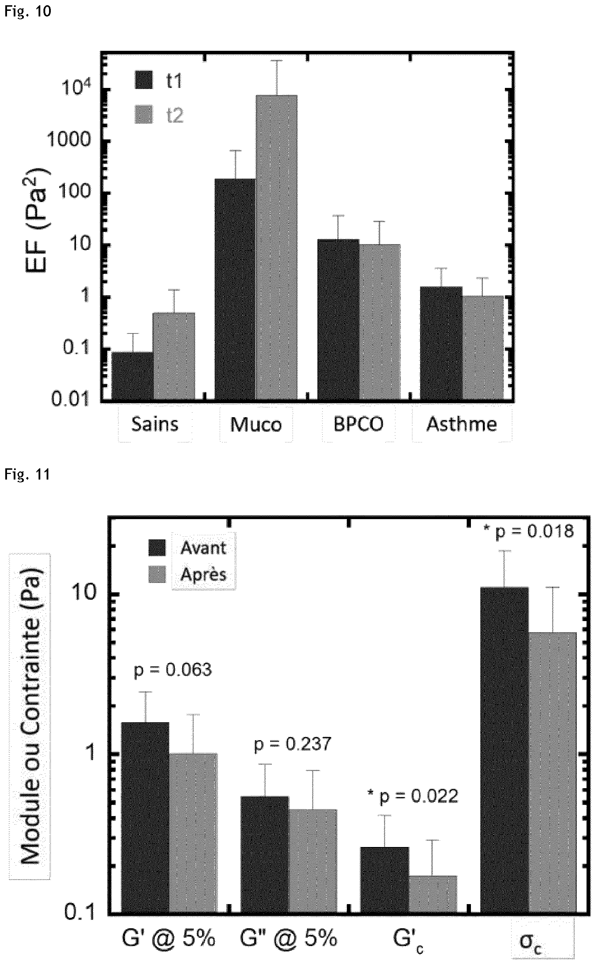

EF est définie par le produit du module d'élasticité G* mesuré dans la zone linéaire (plateau, mesuré à une déformation de 5 %) de la courbe de déformation par la contrainte critique σ_{c}, mesurée au point d'écoulement c de la courbe de déformation. Ces deux paramètres étant individuellement discriminants, leur produit accentue les écarts entre populations. En revanche les variabilités s'ajoutent, la répétabilité moyenne observée sur G' pour les patients atteints de mucoviscidose conduisant à un écart entre t₁ et t₂ (Figure 10 et tableaux 3 et 4).

**Tableau 3: Données à V1 (moyenne ± écart type), Répétition 1, mesurées ou déterminées chez des sujets sains ou atteints de mucoviscidose, de BPCO ou d'asthme.**

| | Sains (n = 11) | Mucoviscidose (n=11) | BPCO (n=11) | Asthme (n=12) |
|---|---|---|---|---|
| G' à 5 % (Pa) | 0.059 ± 0.052 | 1.493 ± 1.517 | 0.768 ± 0.971 | 0.218 ± 0.279 |
| G" à 5 % (Pa) | 0.033 ± 0.028 | 0.533 ± 0.473 | 0.247 ± 0.229 | 0.087 ± 0.109 |
| G* à 5 % (Pa) | 0.068 | 1.585 | 0.807 | 0.235 |
| G_{c} (Pa) | 0.042 ± 0.027 | 0.256 ± 0.182 | 0.202 ± 0.169 | 0.064 ± 0.049 |
| σ_{c} (Pa) | 1.293 ± 1.020 | 11.221 ± 7.271 | 10.574 ± 9.315 | 3.503 ± 2.762 |
| τₚ (Pa) | 0.005 ± 0.004 | 0.337 ± 0.662 | 0.076 ± 0.096 | 0.023 ± 0.035 |
| tan δ (domaine linéaire) | 0.383 ± 0.151 | 0.272 ± 0.053 | 0.284 ± 0.092 | 0.336 ± 0.123 |
| EF (Force Élastique) (Pa²) | 0.088 ± 0.109 | 187.99 ± 475.66 | 12.921 ± 24.072 | 1.584 ± 1.986 |

**Tableau 4: Données à V1 (moyenne ± écart type), Répétition 2, mesurées ou déterminées chez des sujets sains ou atteints de mucoviscidose, de BPCO ou d'asthme.**

| | Sains (n = 11) | Mucoviscidose (n=11) | BPCO (n=11) | Asthme (n=12) |
|---|---|---|---|---|
| G' à 5 % (Pa) | 0.111 ± 0.164 | 1.214 ± 2.499 | 0.610 ± 0.798 | 0.082 ±0.118 |
| G" à 5 % (Pa) | 0.062 ± 0.085 | 0.482 ± 0.959 | 0.221 ± 0.212 | 0.046 ± 0.054 |
| G* à 5 % (Pa) | 0.127 | 1.306 | 0.649 | 0.094 |
| G_{c} (Pa) | 0.045 ± 0.029 | 0.203 ± 0.139 | 0.188 ± 0.125 | 0.068 ± 0.061 |
| σ_{c} (Pa) | 1.587 ± 1.735 | 8.290 ± 4.472 | 9.473 ± 7.857 | 4.768 ± 6.409 |
| τₚ (Pa) | 0.021 ± 0.042 | 16.504 ± 63.646 | 0.038 ± 0.062 | 0.007 ± 0.004 |
| tan δ (domaine linéaire) | 0.338 ± 0.069 | 0.278 ± 0.091 | 0.321 ± 0.113 | 0.419 ± 0.117 |
| EF (Pa²) | 0.496 ± 0.867 | 7692.55 ± 27662.57 | 10.098 ± 18.834 | 1.058 ± 1.261 |

Dans leur ensemble, ces données montrent que les paramètres rhéologiques G', G", G*, σ_{c} τₚ, tan δ et EF, et dans une moindre mesure les paramètres γₚ et γ_{c}, permettent de distinguer nettement et de manière significative les patients souffrant de différentes maladies respiratoires (mucoviscidose, BPCO, ou asthme). Ces paramètres peuvent donc être utilisés comme des biomarqueurs des maladies respiratoires et/ou pulmonaires.

Les données montrent pour la première fois que ces paramètres sont discriminants lorsqu'ils sont mesurés dans la zone linéaire mais également au point d'écoulement c de la courbe de déformation. La combinaison de chacun de ces paramètres, mesurés dans la zone linéaire mais également au point d'écoulement c de la courbe de déformation permet de renforcer la distinction de chacune de ces maladies ainsi que des sujets sains. Ces différents paramètres peuvent donc être utilisés pour un diagnostic, un pronostic, une stratification ou encore le suivi des maladies respiratoires, mais également pour l'évaluation de l'efficacité d'un traitement de ces maladies.

L'évolution des biomarqueurs avant/après traitement des patients atteints de mucoviscidose à la rhDNase (n = 7 patients) et présentée à la Figure 10.

Une réduction (non significative : p > 5 %) est observée sur les modules linéaires G' et G". Une réduction significative (p < 5 %) est observée sur les données G'_{c} et τ_{c} mesurées au point d'écoulement c (Figure 11).

Ces données montrent que le traitement préalable de patients à la rhDNase a un effet sur les paramètres rhéologiques du mucus du patient. Ceci révèle pour la première fois que la mesure de paramètres rhéologiques linéaires et non linéaires permet d'évaluer l'effet mucolytique d'un traitement.

### 2. ETUDE CLINIQUE N°2 : Sujets atteints de DDB, de BPCO ou d'asthme

### 2.1. Matériels et Méthodes

Le protocole clinique a été mené par le CHU de Montpellier. Trois groupes de patients ont été retenus : 24 atteints de dilatation des bronches (DDB), 14 de BPCO et 10 asthmatiques sévères (AS).

Lors d'une visite simple, tous les patients expectorent spontanément. La salive est séparée et l'échantillon est homogénéisé suivant le protocole décrit dans WO/2019/020932 (Method for Processing a Sputum Sample and Method for the Rheometric Measurement of a Preprocessed Sample of This Kind).

Les tests rhéologiques sont réalisés à 37° C avec l'appareil Rheomuco (Rheonova, France). Une séquence d'oscillations à 1 Hz est appliquée avec une amplitude croissante par paliers de 0.1 à 10000 % afin d'extraire les propriétés rhéologiques du sputum dans les domaines linéaire (petites déformations, typiquement < 20 %) et non linéaire (grandes déformations).

### 2.2. Résultats

Les données montrent que les modules G', G", G*, et tan δ (facteur d'amortissement), ainsi que le marqueur σ_{c} (contrainte critique), mesurés au point d'écoulement de la courbe de déformation, sont tous des marqueurs sélectifs, et ce de manière statistiquement significative : les différentes pathologies sont nettement discriminées (avec toutefois un recouvrement BPCO-DDB pour les marqueurs G', G", G* et tan δ ; tableau 5).

Les paramètres G', G" et G*, mesurés dans la zone linéaire de la courbe de déformation (à 5% de déformation), sont particulièrement adaptés pour discriminer les patients atteints d'asthme de ceux atteints de BPCO ou de DDB.

Les données révèlent que le paramètre σ_{c} est particulièrement discriminant, puisqu'il permet de distinguer clairement les sujets atteints de BPCO de ceux atteints de DDB ou encore de ceux atteints d'asthme.

Ainsi, la détermination d'une combinaison d'au moins un paramètre rhéologique dans la zone linéaire de la courbe de déformation (en particulier G', G" ou G*) et d'au moins un paramètre rhéologique dans la zone non linéaire de la courbe de déformation (en particulier au point d'écoulement, notamment σ_{c}), permet de discriminer avec précision chacune des 3 maladies testées (DDB, BPCO, ou asthme).

**Tableau 5: Données rhéologiques mesurées ou déterminées chez des sujets atteints de dilatation des bronches (DDB), de BPCO ou d'asthme (AS).**

| **Paramètre rhéologique** | | **AS** (n=10) | **BPCO** (n=14) | **DDB** (n=24) |
|---|---|---|---|---|
| **Module élastique** | **G' (Pa)** | 12.62±10.06 | 3.01±1.43 | 2.8±1.83 |
| **Module visqueux** | **G" (Pa)** | 2.64±1.74 | 0.85±0.18 | 0.87±0.45 |
| **Module complexe** | **G* (Pa)** | 12.89±10.33 | 3.13±1.42 | 2.93±1.86 |
| **Facteur d'amortissement** | **tan δ (-)** | 0.24±0.06 | 0.34±0.16 | 0.32±0.08 |
| **Contrainte critique** | **σ_{c} (Pa)** | 63.08±62.86 | 24.1±11.92 | 36.19±21.4 |

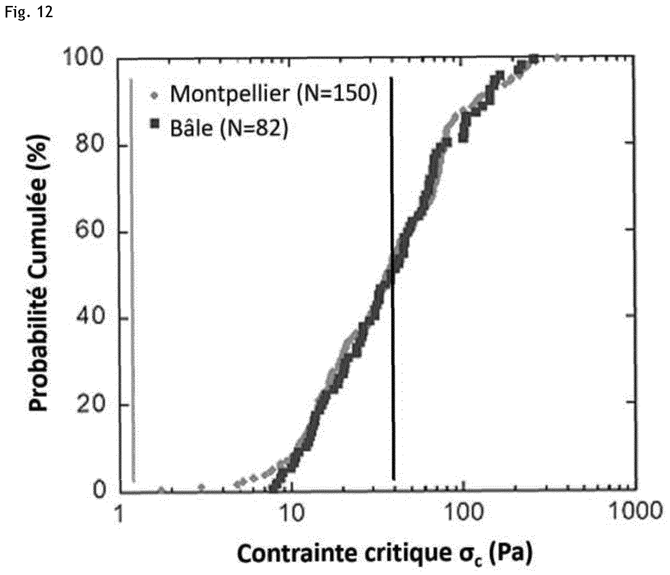

La Figure 12 montre les données obtenues pour le marqueur **σ_{c}** (contrainte critique), pour chaque sujet testé. Les données montrent que des limites de détection peuvent être clairement définie.

La limite de détection des 3 maladies muco-obstructives incluses dans l'étude (DDB, BPCO et asthme, toute maladie confondue) se situe à 1Pa (ligne grise sur la Figure 12) : les maladies muco-obstructive sont détectées si σ_{c}>1Pa (ligne grise sur la Figure 12).

La limite de détection d'une exacerbation (DDB, BPCO et asthme, toute maladie confondue) se situe à 39Pa : une exacerbation est détectée si σ_{c}>39Pa (ligne noire sur la Figure 12).

Dans leur ensemble, ces données montrent que les paramètres rhéologiques G', G", G*, tan δ et σ_{c}, permettent de distinguer nettement et de manière significative les patients souffrant de différentes maladies respiratoires (DDB, BPCO, ou asthme). Ces paramètres peuvent donc être utilisés comme des biomarqueurs des maladies respiratoires et/ou pulmonaires.

Ces données montrent pour la première fois que ces paramètres sont discriminants lorsqu'ils sont mesurés dans la zone linéaire mais également au point d'écoulement c de la courbe de déformation. La combinaison de chacun de ces paramètres, mesurés dans la zone linéaire mais également au point d'écoulement c de la courbe de déformation permet de renforcer la distinction de chacune de ces maladies ainsi que des sujets sains. Ces différents paramètres peuvent donc être utilisés pour un diagnostic, un pronostic, une stratification ou encore le suivi des maladies respiratoires, mais également pour l'évaluation de l'efficacité d'un traitement de ces maladies.

### 3. ETUDE CLINIQUE N°3 : Sujets atteints de BPCO

### 3.1. Matériels et Méthodes

Le protocole clinique a été mené par l'hôpital universitaire de Bâle. Un groupe de patients BPCO a été retenu.

Lors d'une visite simple, tous les patients expectorent spontanément. La salive est séparée et l'échantillon est homogénéisé suivant le protocole décrit dans WO/2019/020932 (Method for Processing a Sputum Sample and Method for the Rheometric Measurement of a Preprocessed Sample of This Kind).

Les tests rhéologiques sont réalisés à 37°C avec l'appareil Rheomuco (Rheonova, France). Une séquence d'oscillations à 1 Hz est appliquée avec une amplitude croissante par paliers de 0.1 à 10000 % afin d'extraire les propriétés rhéologiques du sputum dans les domaines linéaire (petites déformations, typiquement < 20 %) et non linéaire (grandes déformations).

### 3.2. Résultats

Les données montrent les modules G', G", G*, et tan δ (facteur d'amortissement), ainsi que le marqueur σ_{c} (contrainte critique), mesuré au point d'écoulement de la courbe de déformation ; tableau 6).

**Tableau 6: Données rhéologiques mesurées ou déterminées chez des sujets atteints de dilatation des bronches (DDB), de BPCO ou d'asthme (AS).**

| **Paramètre rhéologique** | | **BPCO** (n=50) |
|---|---|---|
| **Module élastique** | **G' (Pa)** | 4.79±4.09 |
| **Module visqueux** | **G" (Pa)** | 1.28±0.78 |
| **Module complexe** | **G* (Pa)** | 5.05±4.14 |
| **Facteur d'amortissement** | **tan** δ (-) | 0.29±0.07 |
| **Contrainte critique** | σ_{c} **(Pa)** | 54.1±26.4 |

La Figure 12 montre les données obtenues pour le marqueur σ_{c} (contrainte critique), pour chaque sujet testé. Les données se corroborent parfaitement avec les données issues du CHU de Montpellier (Etude clinique n°2).

La limite de détection des BPCO se situe à 1Pa (ligne grise sur la Figure 12) : les maladies muco-obstructive sont détectées si σ_{c}>1Pa (ligne grise sur la Figure 12).

La limite de détection d'une exacerbation BPCO se situe à 39Pa : une exacerbation est détectée si σ_{c}>39Pa (ligne noire sur la Figure 12).

### 4. Récapitulatif des 3 études cliniques

Les valeurs moyennes et les écart-types pour chacun des paramètres rhéologiques mesuré ou déterminé dans les trois études cliniques décrites ci-dessus (études n°1 à 3) ont été déterminés, pour chacune des maladies testées et pour les sujets sains. Les valeurs obtenues sont montrées dans les tableaux 1 et 2 présentés ci-dessus, dans la partie descriptive de la présente invention.

Ces données montrent pour la première fois que ces paramètres sont discriminants lorsqu'ils sont mesurés dans la zone linéaire mais également au point d'écoulement c de la courbe de déformation. La combinaison de chacun de ces paramètres, mesurés dans la zone linéaire mais également au point d'écoulement c de la courbe de déformation permet de renforcer la distinction de chacune de ces maladies ainsi que des sujets sains. Ces différents paramètres peuvent donc être utilisés pour un diagnostic, un pronostic, une stratification ou encore le suivi des maladies respiratoires, mais également pour l'évaluation de l'efficacité d'un traitement de ces maladies.

Ainsi, les données des trois études cliniques présentées ci-dessus montrent que la détermination d'une combinaison d'au moins un paramètre rhéologique dans la zone linéaire de la courbe de déformation et d'au moins un paramètre rhéologique dans la zone non linéaire de la courbe de déformation (en particulier au point d'écoulement), permet de discriminer avec précision chacune des 4 maladies testées (mucoviscidose, DDB, BPCO, ou asthme).

## Revendications

1. Méthode *in vitro* de diagnostic, de stratification, de pronostic et/ou de suivi d'une maladie des poumons et/ou une maladie respiratoire, comprenant :
(i) la détermination de la valeur Aₙₗ d'au moins un paramètre rhéologique sur un échantillon de mucus A d'un sujet, dans une zone non linéaire de la courbe de déformation de l'échantillon ;
(ii) la détermination de la valeur Aₗ d'au moins un paramètre rhéologique sur ledit échantillon de mucus A, dans une zone linéaire de la courbe de déformation de l'échantillon ; et
(iii) le diagnostic, la stratification, le pronostic et/ou le suivi de la maladie des poumons et/ou de la maladie respiratoire à partir d'une comparaison desdites valeurs Aₙₗ, Aₗ déterminées avec une valeur limite respective ;
dans laquelle le paramètre rhéologique déterminé dans la zone non linéaire est choisi parmi :
- le module d'élasticité (G'),
- le module de viscosité (G"),
- le facteur d'amortissement (tan δ),
- le module complexe (G*),
- la déformation critique (γ_{c}),
- le seuil de contrainte à l'écoulement (τ_{c}),
- la force élastique (EF),
- la contrainte critique (σ_{c}), et
- une combinaison quelconque de ceux-ci ;
le paramètre rhéologique déterminé dans la zone non linéaire comprenant au moins σ_{c} ; et dans laquelle le paramètre rhéologique déterminé dans la zone linéaire est choisi parmi :
- le module d'élasticité (G'),
- le module de viscosité (G"),
- le facteur d'amortissement (tan δ),
- le module complexe (G*),
- la déformation plastique (γₚ),
- la contrainte plastique(τₚ), et
- une combinaison quelconque de ceux-ci ;
le paramètre rhéologique déterminé dans la zone linéaire comprenant au moins G*.

2. Méthode *in vitro* d'évaluation de l'efficacité d'un traitement d'une maladie des poumons et/ou d'une maladie respiratoire, comprenant :
(i) la détermination de la valeur Aₙₗ d'au moins un paramètre rhéologique sur un échantillon de mucus A d'un sujet souffrant de la maladie des poumons et/ou de la maladie respiratoire et ayant reçu au moins une administration dudit traitement, dans une zone non linéaire de la courbe de déformation de l'échantillon ; et
(ii) la détermination de la valeur Aₗ d'au moins un paramètre rhéologique sur ledit échantillon de mucus A, dans une zone linéaire de la courbe de déformation de l'échantillon ; et
(iii) l'évaluation de l'efficacité du traitement de la maladie des poumons et/ou de la maladie respiratoire à partir d'une comparaison desdites valeurs A_{nl'} Aₗ déterminées avec une valeur limite respective ;
dans laquelle le paramètre rhéologique déterminé dans la zone non linéaire est choisi parmi :
- le module d'élasticité (G'),
- le module de viscosité (G"),
- le facteur d'amortissement (tan δ),
- le module complexe (G*),
- la déformation critique (γ_{c}),
- le seuil de contrainte à l'écoulement (τ_{c}),
- la force élastique (EF),
- la contrainte critique (σ_{c}), et
- une combinaison quelconque de ceux-ci ;
le paramètre rhéologique déterminé dans la zone non linéaire comprenant au moins σ_{c} ; et dans laquelle le paramètre rhéologique déterminé dans la zone linéaire est choisi parmi :
- le module d'élasticité (G'),
- le module de viscosité (G"),
- le facteur d'amortissement (tan δ),
- le module complexe (G*),
- la déformation plastique (γ_{P}),
- la contrainte plastique(σₚ), et
- une combinaison quelconque de ceux-ci ;
le paramètre rhéologique déterminé dans la zone linéaire comprenant au moins G*.

3. Méthode selon la revendication 1 ou 2, dans laquelle la valeur Aₗ d'au moins un paramètre rhéologique est déterminée à une déformation inférieure ou égale à 10%, de préférence à une déformation inférieure ou égale à 5%.

4. Méthode selon l'une quelconque des revendications précédentes, dans laquelle la valeur Aₙₗ d'au moins un paramètre rhéologique est déterminée à une déformation supérieure à 10%.

5. Méthode selon l'une quelconque des revendications précédentes, dans laquelle la valeur Aₙₗ d'au moins un paramètre rhéologique est déterminée au point d'écoulement « c » de l'échantillon de mucus.

6. Méthode selon l'une des revendications précédentes, dans laquelle le sujet est atteint d'une maladie des poumons et/ou d'une maladie respiratoire, ou le pronostic d'une maladie des poumons et/ou d'une maladie respiratoire est négatif, ou une maladie des poumons et/ou une maladie respiratoire est exacerbée, ou le traitement d'une maladie des poumons et/ou d'une maladie respiratoire est inefficace, si au moins une des valeurs Aₗ et/ou Aₙₗ déterminées est différente de la valeur limite respective.

7. Méthode selon l'une quelconque des revendications précédentes, comprenant en outre la détermination de la valeur Bₗ d'au moins un paramètre rhéologique sur un second échantillon de mucus (B) dudit sujet, dans une zone linéaire de la courbe de déformation de l'échantillon ; et/ou la détermination Bₙₗ d'au moins un paramètre rhéologique sur le second échantillon (B), dans une zone non linéaire de la courbe de déformation de l'échantillon ; ledit second échantillon (B) ayant été obtenu/prélevé postérieurement à l'échantillon (A), de préférence ledit échantillon (B) ayant été obtenu au moins 24 heures après l'échantillon (A), de préférence encore au moins 48 heures après l'échantillon (A), de préférence encore au moins 72 heures après l'échantillon (A), de préférence encore au moins 7 jours après l'échantillon (A), de préférence encore au moins 10 jours après l'échantillon (A), de préférence encore au moins 15 jours après l'échantillon (A), de préférence encore au moins 1 mois après l'échantillon (A), de préférence encore au moins 2 mois après l'échantillon (A), de préférence encore au moins 3 mois après l'échantillon (A), de préférence encore ledit échantillon (B) ayant été obtenu entre 7 jours et 6 mois après l'échantillon (A).

8. Méthode selon la revendication 7, comprenant en outre la détermination de l'évolution Eₗ de la valeur du paramètre rhéologique déterminée dans la zone linéaire et/ou l'évolution Eₙₗ de la valeur du paramètre rhéologique déterminée dans la zone non linéaire, dans laquelle l'évolution Eₗ est déterminée en comparant la valeur Bₗ à la valeur Aₗ et/ou l'évolution Eₙₗ est déterminée en comparant la valeur Bₙₗ à la valeur Aₙₗ.

9. Méthode selon la revendication 7 ou 8, dans laquelle le sujet est atteint d'une maladie des poumons et/ou d'une maladie respiratoire, ou le pronostic d'une maladie des poumons et/ou d'une maladie respiratoire est négatif, ou une maladie des poumons et/ou une maladie respiratoire est exacerbée, ou le traitement d'une maladie des poumons et/ou d'une maladie respiratoire est inefficace, si la valeur Bₗ déterminée est différente de la valeur Aₗ déterminée pour au moins un paramètre rhéologique et/ou si la valeur Bₙₗ déterminée est différente de la valeur Aₙₗ déterminée pour au moins un paramètre rhéologique.

10. Méthode selon l'une quelconque des revendications précédentes, dans laquelle le diagnostic, le pronostic et/ou le suivi de la maladie des poumons et/ou de la maladie respiratoire comprennent en outre l'évaluation de l'efficacité d'un traitement de la maladie des poumons et/ou de la maladie respiratoire administré audit sujet.

11. Méthode selon la revendication 10, dans laquelle l'évaluation de l'efficacité du traitement est effectuée selon la méthode de l'une quelconque des revendications 2 à 11, dans laquelle l'évaluation de l'efficacité du traitement est basée sur la valeur (Aₙₗ), la valeur (Aₗ), la valeur (Bₙₗ), la valeur (Bₗ) et une combinaison quelconque de celles-ci ;
de préférence dans laquelle le traitement est efficace, si au moins une des valeurs Aₗ déterminées est inférieure à la valeur Bₗ déterminée pour le même paramètre rhéologique, et/ou si au moins une des valeurs Aₙₗ déterminées est inférieure à la valeur Bₙₗ déterminé pour le même paramètre rhéologique, lorsque :
- le paramètre rhéologique déterminé dans la zone non linéaire comprend au moins un parmi G', G", G*, γ_{c}, τ_{c}, σ_{c}, EF, et une combinaison quelconque de ceux-ci ; et/ou
- le paramètre rhéologique déterminé dans la zone linéaire comprend au moins un parmi G', G", G*, γₚ, τₚ, et une combinaison quelconque de ceux-ci.

12. Méthode selon l'une quelconque des revendications précédentes, dans laquelle la maladie des poumons et/ou la maladie respiratoire est choisie parmi l'amiantose ; l'apnée du sommeil ; le syndrome d'apnées obstructives du sommeil ; l'asphyxie ; l'asthme ; la bronchiectasie ; la bronchite, dont la bronchite aiguë et la bronchite chronique ; la broncho pneumopathie chronique obstructive (BPCO) ; le cancer du poumon ; le cancer bronchique ; le carcinome pulmonaire ; la tumeur pulmonaire ; la coqueluche ; le croup ; le déficit en alpha1-antitrypsine ; l'emphysème ; la mucoviscidose ; la fibrose pulmonaire idiopathique ; la grippe, dont la grippe saisonnière et la grippe A ; le syndrome pulmonaire à hantavirus ; l'hypertension artérielle pulmonaire (HTAP) ; la lymphangioléiomyomatose (LAM) ; la maladie pulmonaire obstructive chronique (MPOC) ; la pleurésie ; la pneumonie ; la bronchopneumonie ; l'embolie pulmonaire ; l'œdème pulmonaire ; l'abcès pulmonaire ; le rhume ; la sinusite ; la sarcoïdose ; le traumatisme thoracique ; la toux chronique ; la tuberculose ; l'infection par le virus respiratoire syncytial (VRS) ; la bronchiolite ; la bronchiolite oblitérante avec organisation pneumonique (BOOP) ; l'allergie, en particulier l'allergie respiratoire ; la rhinite allergique ; la dyskinésie ciliaire primitive (DCP) ; la dilatation des bronches (DDB) ; la pneumoconiose ; le pneumothorax (PNO) ; la pleurésie ; le cancer de la plèvre ; la légionellose ; la psittacose ; de préférence ladite maladie est choisie parmi la DDB, la BPCO, la mucoviscidose et l'asthme.

13. Méthode selon l'une quelconque des revendications précédentes, dans laquelle la valeur (Aₙₗ), la valeur (Aₗ), la valeur (Bₙₗ), et/ou la valeur (Bₗ) sont déterminées à l'aide d'un appareil de mesure rhéologique dans lequel chaque échantillon est de préférence placé entre deux plaques mobiles en rotation l'une par rapport à l'autre, les surfaces en vis-à-vis desdites plaques étant de préférence rugueuses.

## Patentansprüche

1. In-vitro-Verfahren zur Diagnose, Stratifizierung, Prognose und/oder Überwachung einer Erkrankung der Lunge und/oder der Atemwege, umfassend:
(i) die Bestimmung des Wertes Aₙₗ von mindestens einem rheologischen Parameter an einer Schleimprobe A eines Probanden in einem nichtlinearen Bereich der Verformungskurve der Probe;
(ii) die Bestimmung des Wertes Aₗ von mindestens einem rheologischen Parameter an der Schleimprobe A in einem linearen Bereich der Verformungskurve der Probe; und
(iii) die Diagnose, die Stratifizierung, die Prognose und/oder die Überwachung der Erkrankung der Lunge und/oder der Atemwege auf der Basis eines Vergleichs der bestimmten Werte Aₙₗ, Aₗ mit einem jeweiligen Grenzwert;
wobei der im nichtlinearen Bereich bestimmte rheologische Parameter ausgewählt ist aus:
- dem Elastizitätsmodul (G'),
- dem Viskositätsmodul (G"),
- dem Dämpfungsfaktor (tan δ),
- dem Komplexmodul (G*),
- der kritischen Verformung (γ_{c}),
- dem Schwellenwert für die Fließspannung (τ_{c}),
- der elastischen Kraft (EF),
- der kritischen Spannung (σ_{c}), und
- einer beliebigen Kombination davon;
wobei der im nichtlinearen Bereich bestimmte rheologische Parameter mindestens σ_{c} umfasst; und
wobei der im linearen Bereich bestimmte rheologische Parameter ausgewählt ist aus:
- dem Elastizitätsmodul (G'),
- dem Viskositätsmodul (G"),
- dem Dämpfungsfaktor (tan δ),
- dem Komplexmodul (G*),
- der plastischen Verformung (γₚ),
- der plastischen Spannung (τₚ), und
- einer beliebigen Kombination davon;
wobei der im linearen Bereich bestimmte rheologische Parameter mindestens G* umfasst.

2. In-vitro-Verfahren zur Bewertung der Wirksamkeit einer Behandlung einer Erkrankung der Lunge und/oder der Atemwege, umfassend:
(i) die Bestimmung des Wertes Aₙ₁ von mindestens einem rheologischen Parameter an einer Schleimprobe A eines Probanden, der an der Erkrankung der Lunge und/oder der Atemwege leidet und mindestens eine Verabreichung der Behandlung erhalten hat, in einem nichtlinearen Bereich der Verformungskurve der Probe; und
(ii) die Bestimmung des Wertes Aₗ von mindestens einem rheologischen Parameter an der Schleimprobe A in einem linearen Bereich der Verformungskurve der Probe; und
(iii) die Bewertung der Wirksamkeit einer Behandlung der Erkrankung der Lunge und/oder der Atemwege auf der Basis eines Vergleichs der bestimmten Werte Aₙₗ, Aₗ mit einem jeweiligen Grenzwert;
wobei der im nichtlinearen Bereich bestimmte rheologische Parameter ausgewählt ist aus:
- dem Elastizitätsmodul (G'),
- dem Viskositätsmodul (G"),
- dem Dämpfungsfaktor (tan δ),
- dem Komplexmodul (G*),
- der kritischen Verformung (γ_{c}),
- dem Schwellenwert für die Fließspannung (γ_{c}),
- der elastischen Kraft (EF),
- der kritischen Spannung (σ_{c}), und
- einer beliebigen Kombination davon;
wobei der im nichtlinearen Bereich bestimmte rheologische Parameter mindestens σ_{c} umfasst; und
wobei der im linearen Bereich bestimmte rheologische Parameter ausgewählt ist aus:
- dem Elastizitätsmodul (G'),
- dem Viskositätsmodul (G"),
- dem Dämpfungsfaktor (tan δ),
- dem Komplexmodul (G*),
- der plastischen Verformung (γₚ),
- der plastischen Spannung (iₚ), und
- einer beliebigen Kombination davon;
wobei der im linearen Bereich bestimmte rheologische Parameter mindestens G* umfasst.

3. Verfahren nach Anspruch 1 oder 2, wobei der Wert Aₗ von mindestens einem rheologischen Parameter bei einer Verformung von weniger als oder gleich 10%, vorzugsweise bei einer Verformung von weniger als oder gleich 5%, bestimmt wird.

4. Verfahren nach einem der vorhergehenden Ansprüche, wobei der Wert Aₙₗ von mindestens einem rheologischen Parameter bei einer Verformung von mehr als 10% bestimmt wird.

5. Verfahren nach einem der vorhergehenden Ansprüche, wobei der Wert Aₙₗ von mindestens einem rheologischen Parameter am Fließpunkt "c" der Schleimprobe bestimmt wird.

6. Verfahren nach einem der vorhergehenden Ansprüche, wobei der Proband an einer Erkrankung der Lunge und/oder der Atemwege leidet oder die Prognose einer Erkrankung der Lunge und/oder der Atemwege negativ ist oder eine Erkrankung der Lunge und/oder der Atemwege exazerbiert ist oder die Behandlung einer Erkrankung der Lunge und/oder der Atemwege unwirksam ist, wenn mindestens einer der bestimmten Werte Aₗ und/oder Aₙₗ von dem jeweiligen Grenzwert unterscheidet.

7. Verfahren nach einem der vorhergehenden Ansprüche, ferner umfassend die Bestimmung des Wertes Bₗ von mindestens einem rheologischen Parameter an einer zweiten Schleimprobe (B) des Probanden in einem linearen Bereich der Verformungskurve der Probe; und/oder die Bestimmung Bₙₗ von mindestens einem rheologischen Parameter an der zweiten Probe (B) in einem nichtlinearen Bereich der Verformungskurve der Probe; wobei die zweite Probe (B) nach der Probe (A) erhalten/entnommen wurde, wobei die Probe (B) vorzugsweise mindestens 24 Stunden nach der Probe (A) erhalten wurde, noch vorzugsweise mindestens 48 Stunden nach der Probe (A), noch vorzugsweise mindestens 72 Stunden nach der Probe (A), noch vorzugsweise mindestens 7 Tage nach der Probe (A), noch vorzugsweise mindestens 10 Tage nach der Probe (A), noch vorzugsweise mindestens 15 Tage nach der Probe (A), noch vorzugsweise mindestens 1 Monat nach der Probe (A), noch vorzugsweise mindestens 2 Monate nach der Probe (A), noch vorzugsweise mindestens 3 Monate nach der Probe (A), wobei die Probe (B) noch vorzugsweise zwischen 7 Tagen und 6 Monaten nach der Probe (A) erhalten wurde.

8. Verfahren nach Anspruch 7, ferner umfassend die Bestimmung der Entwicklung Eₗ des im linearen Bereich bestimmten Werts des rheologischen Parameters und/oder der Entwicklung Eₙₗ des im nichtlinearen Bereich bestimmten Werts des rheologischen Parameters, wobei die Entwicklung Eₗ durch Vergleich des Werts Bₗ mit dem Wert Aₗ bestimmt wird und/oder die Entwicklung von Eₙₗ durch Vergleich des Werts Bₙₗ mit dem Wert Aₙₗ bestimmt wird.

9. Verfahren nach Anspruch 7 oder 8, wobei der Proband an einer Erkrankung der Lunge und/oder der Atemwege leidet, oder die Prognose einer Erkrankung der Lunge und/oder der Atemwege negativ ist, oder eine Erkrankung der Lunge und/oder der Atemwege exazerbiert ist, oder die Behandlung einer Erkrankung der Lunge und/oder der Atemwege unwirksam ist, wenn sich der bestimmte Wert Bₗ von dem für mindestens einen rheologischen Parameter bestimmten Wert Aₗ unterscheidet und/oder wenn sich der bestimmte Wert Bₙₗ von dem für mindestens einen rheologischen Parameter bestimmten Wert Aₙₗ unterscheidet.

10. Verfahren nach einem der vorhergehenden Ansprüche, wobei die Diagnose, die Prognose und/oder die Überwachung der Erkrankung der Lunge und/oder der Atemwege ferner die Bewertung der Wirksamkeit einer Behandlung der Erkrankung der Lunge und/oder der Atemwege umfasst, die dem Probanden verabreicht wurde.

11. Verfahren nach Anspruch 10, wobei die Bewertung der Wirksamkeit der Behandlung nach dem Verfahren nach einem der Ansprüche 2 bis 11 durchgeführt wird, wobei die Bewertung der Wirksamkeit der Behandlung auf dem Wert (Aₙₗ), dem Wert (Aₗ), dem Wert (Bₙₗ), dem Wert (Bₗ) und einer beliebigen Kombination davon basiert;
wobei vorzugweise die Behandlung wirksam ist, wenn mindestens einer der bestimmten Werte Aₗ niedriger als der bestimmte Wert Bₗ für denselben rheologischen Parameter ist, und/oder wenn mindestens einer der bestimmten Werte Aₙₗ niedriger als der bestimmte Wert Bₙₗ für denselben rheologischen Parameter ist, wenn:
- der bestimmte rheologische Parameter im nichtlinearen Bereich mindestens einen Parameter von G', G", G*, γ_{c}, τ_{c}, σ_{c}, EF und eine beliebige Kombination davon umfasst; und/oder
- der bestimmte rheologische Parameter im linearen Bereich mindestens einen Parameter von G', G", G*, γₚ, τₚ und eine beliebige Kombination davon umfasst.

12. Verfahren nach einem der vorhergehenden Ansprüche, wobei die Erkrankung der Lunge und/oder Atemwege ausgewählt ist aus Asbestose; Schlafapnoe; dem obstruktiven Schlafapnoe-Syndrom; Asphyxie; Asthma; Bronchiektasie; Bronchitis, akute Bronchitis und chronische Bronchitis eingeschlossen; chronisch obstruktiver Broncho-Pneumopathie (COBD); Lungenkrebs; Bronchialkrebs; Lungenkarzinom; Lungentumor; Keuchhusten; Krupp; Alphal-Antitrypsin-Mangel; Emphysem; Mukoviszidose; idiopathischer Lungenfibrose; Grippe, saisonale Grippe und Influenza A eingeschlossen; Hantavirus-Lungensyndrom; pulmonaler arterieller Hypertonie (PAH); Lymphangioleiomyomatose (LAM); chronisch obstruktiver Lungenerkrankung (COPD); Pleuritis; Pneumonie; Bronchopneumonie; Lungenembolie; Lungenödem; Lungenabszess; Schnupfen; Sinusitis; Sarkoidose; Thoraxtrauma; chronischem Husten; Tuberkulose; Infektion mit dem Respiratory Syncytial Virus (RSV); Bronchiolitis; Bronchiolitis obliterans mit pneumonischer Organisation (BOOP); Allergie, insbesondere respiratorischer Allergie; allergischer Rhinitis; primärer ziliärer Dyskinesie (PCD); Bronchialdilatation (BDD); Pneumokoniose; Pneumothorax (PNO); Pleuritis; Pleurakrebs; Legionellose; Psittakose; vorzugsweise ist die genannte Krankheit ausgewählt aus BDD, COBD, Mukoviszidose und Asthma.

13. Verfahren nach einem der vorhergehenden Ansprüche, wobei der Wert (Aₙₗ), der Wert (Aₗ), der Wert (Bₙₗ) und/oder der Wert (Bₗ) mithilfe eines rheologischen Messgeräts bestimmt werden, in dem jede Probe vorzugsweise zwischen zwei Platten angeordnet ist, die relativ zueinander drehbeweglich sind, wobei die einander zugewandten Oberflächen der Platten vorzugsweise rau sind.

## Claims

1. An *in-vitro* method for diagnosing, stratifying, prognosing and/or monitoring a lung disease and/or a respiratory disease, comprising:
(i) determining the value Aₙₗ of at least one rheological parameter on a sample of mucus A of a subject, in a non-linear region of the stress-strain curve of the sample;
(ii) determining the value Aₗ of at least one rheological parameter on said sample of mucus A, in a linear region of the stress-strain curve of the sample; and
(iii) diagnosing, stratifying, prognosing and/or monitoring lung disease and/or respiratory disease from a comparison of said values Aₙₗ, Aₗ determined with a respective limit value;
wherein the rheological parameter determined in the non-linear region is selected from
- the elastic modulus (G'),
- the viscose modulus (G"),
- the damping factor (tan δ),
- the complex modulus (G*),
- the critical deformation (γ_{c}),
- the flow stress threshold (τ_{c}),
- the elastic force (EF),
- the critical stress (σ_{c}), and
- any combination thereof;
the rheological parameter determined in the nonlinear region comprising at least σ_{c}; and
wherein the rheological parameter determined in the linear region is selected from:
- the elastic modulus (G'),
- the viscose modulus (G"),
- the damping factor (tan δ),
- the complex modulus (G*),
- the plastic deformation (γₚ),
- the plastic stress (τₚ), and
- any combination thereof;
the rheological parameter determined in the linear region comprising at least G*.

2. An *in-vitro* method for evaluating the efficacy of a treatment of a lung disease and/or a respiratory disease, comprising:
(i) determining the value Aₙₗ of at least one rheological parameter on a sample of mucus A of a subject with lung disease and/or respiratory disease and having received at least one administration of said treatment, in a non-linear region of the stress-strain curve of the sample; and
(ii) determining the value Aₗ of at least one rheological parameter on said sample of mucus A, in a linear region of the stress-strain curve of the sample; and
(iii) evaluating the efficacy of the treatment of lung disease and/or respiratory disease from a comparison of said values Aₙₗ, Aₗ determined with a respective limit value;
wherein the rheological parameter determined in the non-linear region is selected from
- the elastic modulus (G'),
- the viscose modulus (G"),
- the damping factor (tan δ),
- the complex modulus (G*),
- the critical deformation (γ_{c}),
- the flow stress threshold (τ_{c}),
- the elastic force (EF),
- the critical stress (σ_{c}), and
- any combination thereof;
the rheological parameter determined in the nonlinear region comprising at least σ_{c}; and
wherein the rheological parameter determined in the linear region is selected from:
- the elastic modulus (G'),
- the viscose modulus (G"),
- the damping factor (tan δ),
- the complex modulus (G*),
- the plastic deformation (γₚ),
- the plastic stress(τₚ), and
- any combination thereof;
the rheological parameter determined in the linear region comprising at least G*.

3. The method according to claim 1 or 2, wherein the value Aₗ of the at least one rheological parameter is determined at a deformation less than or equal to 10%, preferably less than or equal to 5%.

4. The method according to any one of the preceding claims, wherein the Aₙₗ value of at least one rheological parameter is determined at a deformation greater than 10%.

5. The method according to any one of the preceding claims, wherein the Aₙₗ value of at least one rheological parameter is determined at the flow point "c" of the mucus sample.

6. The method according to any one of the preceding claims, wherein the subject has a lung disease and/or a respiratory disease, or the prognosis of a lung disease and/or a respiratory disease is negative, or a lung disease and/or a respiratory disease is exacerbated, or the treatment of a lung disease and/or a respiratory disease is ineffective, if at least one of the determined values Aₗ and/or Aₙₗ is different from the respective limit value.

7. The method according to any one of the preceding claims, further comprising determining the value Bₗ of at least one rheological parameter on a second mucus sample (B) of said subject, in a linear region of the stress-strain curve of the sample; and/or determining the value Bₙₗ of at least one rheological parameter on the second sample (B) in a non-linear region of the stress-strain curve of the sample, said second sample (B) having been obtained/taken after sample (A), preferably, said sample (B) having been obtained at least 24 hours after sample (A), more preferably at least 48 hours after sample (A), more preferably at least 72 hours after sample (A), more preferably at least 7 days after sample (A), more preferably at least 10 days after sample (A), more preferably at least 15 days after sample (A), more preferably at least 1 month after sample (A), more preferably at least 2 months after sample (A), more preferably at least 3 months after sample (A), more preferably, said sample (B) having been obtained between 7 days and 6 months after sample (A).

8. The method according to claim 7, further comprising the determination of the evolution Eₗ of the value of the rheological parameter determined in the linear region and/or the evolution Eₙₗ of the value of the rheological parameter determined in the non-linear region, wherein the evolution Eₗ is determined by comparing the value Bₗ with the value Aₗ and/or the evolution Eₙₗ is determined by comparing the value Bₙₗ with the value Aₙₗ.

9. The method according to claim 7 or 8, wherein the subject has a lung disease and/or a respiratory disease, or the prognosis of a lung disease and/or a respiratory disease is negative, or a lung disease and/or a respiratory disease is exacerbated, or the treatment of a lung disease and/or a respiratory disease is ineffective, if the value Bₗ determined is different from the value Aₗ determined for at least one rheological parameter and/or if the value determined Bₙₗ is different from the value Aₙₗ determined for at least one rheological parameter.

10. The method according to any one of the preceding claims, wherein the diagnosis, the prognosis and/or the monitoring of lung disease and/or respiratory disease further comprise the evaluation of the efficacy of a treatment of lung disease and/or respiratory disease administered to said subject.

11. The method according to claim 10, wherein the evaluation of the efficacy of the treatment is carried out according to the method of any one of claims 2 to 11, wherein the evaluation of the efficacy of the treatment is based on the value (Aₙₗ), the value (Aₗ), the value (Bₙₗ), the value (Bₗ) and any combination thereof;
preferably, wherein the treatment is effective, if at least one of the values Aₗ determined is less than the value Bₗ determined for the same rheological parameter, and/or if at least one of the values Aₙₗ determined is less than the value Bₙₗ determined for the same rheological parameter, when:
- the rheological parameter determined in the nonlinear region comprises at least one of G', G", G*, γ_{c}, τ_{c}, σ_{c}, EF, and any combination thereof; and/or
- the rheological parameter determined in the linear region comprises at least one of G', G", G*, γₚ, τₚ, and any combination thereof.

12. The method according to any one of the preceding claims, wherein the lung disease and/or respiratory disease is selected from asbestosis; sleep apnoea; obstructive sleep apnoea syndrome; asphyxia; asthma; bronchiectasis; bronchitis, including acute bronchitis and chronic bronchitis; chronic obstructive pulmonary disease (COPD); lung cancer; bronchial cancer; lung carcinoma; lung tumour; pertussis; croup; alpha-1-antitrypsin deficiency; emphysema; cystic fibrosis; idiopathic pulmonary fibrosis; influenza, including seasonal influenza and influenza A; hantavirus pulmonary syndrome; pulmonary arterial hypertension (PAH); lymphangioleiomyomatosis (AML); chronic obstructive pulmonary disease (COPD); pleurisy; pneumonia; bronchopneumonia; pulmonary embolism; pulmonary oedema; pulmonary abscess; cold; sinusitis; sarcoidosis; chest trauma; chronic cough; tuberculosis; respiratory syncytial virus (RSV) infection; bronchiolitis; bronchiolitis obliterans organizing pneumonia (BOOP); allergy, especially respiratory allergy; allergic rhinitis; primary ciliary dyskinesia (PCD); bronchial dilatation (BDD); pneumoconiosis; pneumothorax (PNX); pleurisy; pleural cancer; legionellosis; psittacosis; preferably said disease is selected from BDD, COPD, cystic fibrosis and asthma.

13. The method according to any one of the preceding claims, wherein the value (Aₙₗ), the value (Aₗ), the value (Bₙₗ), and/or the value (Bₗ) are determined by means of a rheological measuring apparatus in which each sample is preferably placed between two plates rotatable relative to each other and the facing surfaces of said plates are preferably rough.
